(19)

Europäisches
Patentamt
European
Patent Office
Office européen
des brevets

(11) **EP 4 497 759 A1**

(12) **EUROPEAN PATENT APPLICATION**
published in accordance with Art. 153(4) EPC

(43) Date of publication:
**29.01.2025 Bulletin 2025/05**

(21) Application number: 23740060.1

(22) Date of filing: **12.01.2023**

(51) International Patent Classification (IPC):
*C07K 16/46* (2006.01)      *C12N 15/13* (2006.01)
*A61K 39/395* (2006.01)      *A61P 35/00* (2006.01)

(52) Cooperative Patent Classification (CPC):
**A61K 39/395; A61P 35/00; C07K 16/00;
C07K 16/46**

(86) International application number:
**PCT/CN2023/071869**

(87) International publication number:
**WO 2023/134718 (20.07.2023 Gazette 2023/29)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **14.01.2022  CN 202210044655**

(71) Applicant: **Oricell Therapeutics Co., Ltd.
Shanghai 201203 (CN)**

(72) Inventors:
• **HE, Xiaowen
Shanghai 201203 (CN)**

• **ZHOU, Jincai
Shanghai 201203 (CN)**
• **SHI, Zhongjun
Shanghai 201203 (CN)**
• **CHEN, Siye
Shanghai 201203 (CN)**
• **YANG, Yue
Shanghai 201203 (CN)**

(74) Representative: **Weickmann & Weickmann
PartmbB
Postfach 860 820
81635 München (DE)**

Remarks:
The complete document including Reference
Table(s) and the Sequence Listing(s) can be
downloaded from the EPO website

(54) **CHIMERIC ANTIGEN RECEPTOR TARGETING GPRC5D AND APPLICATION THEREOF**

(57)    The present application relates to a chimeric antigen receptor, comprising a first antigen binding domain and a second antigen binding domain. The first antigen binding domain targets a G protein-coupled receptor class C group 5 member D (GPRC5D) protein, and the second antigen binding domain targets the GPRC5D protein. The present application further provides a cell comprising the chimeric antigen receptor and use thereof.

EP 4 497 759 A1

## Description

**Field of the Invention**

The present application relates to the field of biomedicine, and particularly to a novel anti-GPRC5D chimeric antigen receptor, a cell comprising the chimeric antigen receptor, as well as use thereof in the treatment of tumors.

**Background of the Invention**

Multiple myeloma (MM) is the second most common hematologic malignancy and ranks second in the cancer mortality rate. MM is a kind of plasma cell malignancy, often accompanied by multiple osteolytic lesions, renal function impairments, bone marrow infiltration, hypercalcemia, and anemia. The current main treatment for MM is systemic chemotherapy with severe side effects, and complete cure cannot be achieved.

G protein-coupled receptor class C group 5 member D (GPRC5D) protein is an atypical surface orphan receptor. GPRCD5, like other C5 family receptors, has a short amino terminus and is therefore conformationally very similar to the C4 family. Its expression in normal tissues is restricted to hair follicles, but it is also specifically highly expressed in the bone marrow of MM patients and is highly correlated with plasma cell tumor burden and genetic aberrations.

With the introduction of novel drugs such as protease inhibitors (PIs), immunomodulatory drugs (IMiDs), and monoclonal antibodies (mAbs), the treatment options for MM have been expanded. However, MM still exhibits a very high recurrence rate and develops drug resistance. Chimeric antigen receptor T cell (CAR-T) therapies have produced significant efficacy and controllable toxicity in the treatment of MM. Among them, the mainstream CAR-T cell therapy targeting B-cell maturation antigens (BCMAs) presents a target escape problem for MM patients with BCMA-negative or low BCMA expression. The specifically high expression of GPRC5D on plasma cells makes it an ideal therapeutic target for MM, but drug therapy products targeting the GPRC5D are not yet mature.

**Summary of the Invention**

The present application provides a chimeric antigen receptor, comprising two antigen binding domains, both of which are capable of targeting GPRC5D. The present application further provides a cell comprising the chimeric antigen receptor. The cell has one or more of the following properties: (1) strong amplification ability, (2) being capable of killing GPRC5D-expressing target cells, (3) secreting cytokines under the stimulation of target cells, (4) strong proliferation ability under the stimulation of target cells, (4) inhibiting the tumor growth, and (5) good safety.

In one aspect, the present application provides a chimeric antigen receptor, comprising a first antigen binding domain and a second antigen binding domain, the first antigen binding domain targets G protein-coupled receptor class C group 5 member D (GPRC5D) protein, and the second antigen binding domain targets GPRC5D protein.

In some embodiments, the first antigen binding domain and/or the second antigen binding domain of the chimeric antigen receptor comprise an antibody or an antigen binding fragment thereof.

In some embodiments, the antigen binding fragment comprises Fab, Fab', F(ab)$_2$, an Fv fragment, F(ab')$_2$, scFv, di-scFv, VHH and/or dAb.

In some embodiments, the antigen binding fragment comprises a VHH.

In some embodiments, the first antigen binding domain of the chimeric antigen receptor comprises at least one CDR of antibody heavy chain variable region VH, the VH comprises an amino acid sequence as set forth in any one of SEQ ID NO: 15, SEQ ID NO: 19, SEQ ID NO: 24 and SEQ ID NO: 30.

In some embodiments, the first antigen binding domain of the chimeric antigen receptor comprises a HCDR3, and the HCDR3 comprises an amino acid sequence as set forth in SEQ ID NO: 33.

In some embodiments, the first antigen binding domain of the chimeric antigen receptor comprises a HCDR3, and the HCDR3 comprises an amino acid sequence as set forth in any one of SEQ ID NO: 10, SEQ ID NO: 18, SEQ ID NO: 22 and SEQ ID NO: 27.

In some embodiments, the first antigen binding domain of the chimeric antigen receptor comprises a HCDR2, and the HCDR2 comprises an amino acid sequence as set forth in SEQ ID NO: 34 (IX$_1$X$_2$X$_3$X$_4$GX$_5$T, wherein X$_1$ is N or T, X$_2$ is S or W, X$_3$ is G or S, X$_4$ is D or G, and X$_5$ is N, S or T).

In some embodiments, the first antigen binding domain of the chimeric antigen receptor comprises a HCDR2, and the HCDR2 comprises an amino acid sequence as set forth in any one of SEQ ID NO: 9, SEQ ID NO: 17, SEQ ID NO: 21 and SEQ ID NO: 26.

In some embodiments, the first antigen binding domain of the chimeric antigen receptor comprises a HCDR1, and the HCDR1 comprises an amino acid sequence as set forth in SEQ ID NO: 35.

In some embodiments, the first antigen binding domain of the chimeric antigen receptor comprises a HCDR1, and the HCDR1 comprises an amino acid sequence as set forth in any one of SEQ ID NO: 8, SEQ ID NO: 16, SEQ ID NO:

20 and SEQ ID NO: 25.

In some embodiments, the first antigen binding domain of the chimeric antigen receptor comprises a HCDR1, a HCDR2 and a HCDR3, the HCDR1 comprises an amino acid sequence as set forth in SEQ ID NO: 35, the HCDR2 comprises an amino acid sequence as set forth in SEQ ID NO: 34 ($IX_1X_2X_3X_4GX_5T$, wherein $X_1$ is N or T, $X_2$ is S or W, $X_3$ is G or S, $X_4$ is D or G, and $X_5$ is N, S or T), and the HCDR3 comprises an amino acid sequence as set forth in SEQ ID NO: 33.

In some embodiments, the first antigen binding domain of the chimeric antigen receptor comprises a HCDR1, a HCDR2 and a HCDR3, and the HCDR1, HCDR2, HCDR3 comprise any one group of amino acid sequences selected from:

(1) the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10;

(2) the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18;

(3) the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22; and

(4) the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27.

In some embodiments, the first antigen binding domain of the chimeric antigen receptor comprises a H-FR1, a C-terminal of the H-FR1 is connected to an N-terminal of the HCDR1 directly or indirectly, and the H-FR1 comprises an amino acid sequence as set forth in SEQ ID NO: 11.

In some embodiments, the first antigen binding domain of the chimeric antigen receptor comprises a H-FR2, the H-FR2 is located between the HCDR1 and the HCDR2, and the H-FR2 comprises an amino acid sequence as set forth in any one of SEQ ID NO: 12, SEQ ID NO: 28 and SEQ ID NO: 23.

In some embodiments, the first antigen binding domain of the chimeric antigen receptor comprises a H-FR3, the H-FR3 is located between the HCDR2 and the HCDR3, and the H-FR3 comprises an amino acid sequence as set forth in SEQ ID NO: 13 or SEQ ID NO: 29.

In some embodiments, the first antigen binding domain of the chimeric antigen receptor comprises a H-FR4, an N-terminal of the H-FR4 is connected to a C-terminal of the HCDR3, and the H-FR4 comprises an amino acid sequence as set forth in SEQ ID NO: 14.

In some embodiments, the first antigen binding domain of the chimeric antigen receptor comprises a VH, and the VH comprises an amino acid sequence as set forth in any one of SEQ ID NO: 15, SEQ ID NO: 19, SEQ ID NO: 24 and SEQ ID NO: 30.

In some embodiments, the first antigen binding domain of the chimeric antigen receptor is a VHH, and the VHH comprises an amino acid sequence as set forth in any one of SEQ ID NO: 15, SEQ ID NO: 19, SEQ ID NO: 24 and SEQ ID NO: 30.

In some embodiments, the second antigen binding domain of the chimeric antigen receptor comprises at least one CDR of antibody heavy chain variable region VH, the VH comprises an amino acid sequence as set forth in any one of SEQ ID NO: 15, SEQ ID NO: 19, SEQ ID NO: 24 and SEQ ID NO: 30.

In some embodiments, the second antigen binding domain of the chimeric antigen receptor comprises a HCDR3, and the HCDR3 comprises an amino acid sequence as set forth in SEQ ID NO: 33.

In some embodiments, the second antigen binding domain of the chimeric antigen receptor comprises a HCDR3, and the HCDR3 comprises an amino acid sequence as set forth in any one of SEQ ID NO: 10, SEQ ID NO: 18, SEQ ID NO: 22 and SEQ ID NO: 27.

In some embodiments, the second antigen binding domain of the chimeric antigen receptor comprises a HCDR2, and the HCDR2 comprises an amino acid sequence as set forth in SEQ ID NO: 34 ($IX_1X_2X_3X_4GX_5T$, wherein $X_1$ is N or T, $X_2$ is S or W, $X_3$ is G or S, $X_4$ is D or G, and $X_5$ is N, S or T).

In some embodiments, the second antigen binding domain of the chimeric antigen receptor comprises a HCDR2, and the HCDR2 comprises an amino acid sequence as set forth in any one of SEQ ID NO: 9, SEQ ID NO: 17, SEQ ID NO: 21 and SEQ ID NO: 26.

In some embodiments, the second antigen binding domain of the chimeric antigen receptor comprises a HCDR1, and the HCDR1 comprises an amino acid sequence as set forth in SEQ ID NO: 35.

In some embodiments, the second antigen binding domain of the chimeric antigen receptor comprises a HCDR1, and the HCDR1 comprises an amino acid sequence as set forth in any one of SEQ ID NO: 8, SEQ ID NO: 16, SEQ ID NO:

20 and SEQ ID NO: 25.

In some embodiments, the second antigen binding domain of the chimeric antigen receptor comprises a HCDR1, a HCDR2 and a HCDR3, the HCDR1 comprises an amino acid sequence as set forth in SEQ ID NO: 35, the HCDR2 comprises an amino acid sequence as set forth in SEQ ID NO: 34 ($IX_1X_2X_3X_4GX_5T$, wherein $X_1$ is N or T, $X_2$ is S or W, $X_3$ is G or S, $X_4$ is D or G, and $X_5$ is N, S or T), and the HCDR3 comprises an amino acid sequence as set forth in SEQ ID NO: 33.

In some embodiments, the second antigen binding domain of the chimeric antigen receptor comprises a HCDR1, a HCDR2 and a HCDR3, and the HCDR1, HCDR2, HCDR3 comprise any one group of amino acid sequences selected from:

(1) the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10;

(2) the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18;

(3) the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22; and

(4) the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27.

In some embodiments, the second antigen binding domain of the chimeric antigen receptor comprises a H-FR1, a C-terminal of the H-FR1 is connected to an N-terminal of the HCDR1 directly or indirectly, and the H-FR1 comprises an amino acid sequence as set forth in SEQ ID NO: 11.

In some embodiments, the second antigen binding domain of the chimeric antigen receptor comprises a H-FR2, the H-FR2 is located between the HCDR1 and the HCDR2, and the H-FR2 comprises an amino acid sequence as set forth in any one of SEQ ID NO: 12, SEQ ID NO: 28 and SEQ ID NO: 23.

In some embodiments, the second antigen binding domain of the chimeric antigen receptor comprises a H-FR3, the H-FR3 is located between the HCDR2 and the HCDR3, and the H-FR3 comprises an amino acid sequence as set forth in SEQ ID NO: 13 or SEQ ID NO: 29.

In some embodiments, the second antigen binding domain of the chimeric antigen receptor comprises a H-FR4, an N-terminal of the H-FR4 is connected to a C-terminal of the HCDR3, and the H-FR4 comprises an amino acid sequence as set forth in SEQ ID NO: 14.

In some embodiments, the second antigen binding domain of the chimeric antigen receptor comprises a VH, and the VH comprises an amino acid sequence as set forth in any one of SEQ ID NO: 15, SEQ ID NO: 19, SEQ ID NO: 24 and SEQ ID NO: 30.

In some embodiments, the second antigen binding domain of the chimeric antigen receptor is a VHH, and the VHH comprises an amino acid sequence as set forth in any one of SEQ ID NO: 15, SEQ ID NO: 19, SEQ ID NO: 24 and SEQ ID NO: 30.

In some embodiments, the first antigen binding domain and the second antigen binding domain of the chimeric antigen receptor comprise a HCDR1, a HCDR2 and a HCDR3, respectively, and the sequences of the HCDR1, the HCDR2 and the HCDR3 of the first antigen binding domain and the second antigen binding domain are selected from any one group of amino acid sequences below:

(1) in the first antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10; in the second antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10;

(2) in the first antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10; in the second antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18;

(3) in the first antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10; in the second antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22;

(4) in the first antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10; in the second antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27;

(5) in the first antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18; in the second antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10;

(6) in the first antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18; in the second antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18;

(7) in the first antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18; in the second antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22;

(8) in the first antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18; in the second antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27;

(9) in the first antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22; in the second antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10;

(10) in the first antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22; in the second antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18;

(11) in the first antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22; in the second antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22;

(12) in the first antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22; in the second antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27;

(13) in the first antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27; in the second antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10;

(14) in the first antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27; in the second antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18;

(15) in the first antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27; in the second antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22; and

(16) in the first antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27; in the second antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27.

In some embodiments, the first antigen binding domain and the second antigen binding domain of the chimeric antigen receptor target the same epitope of GPRC5D.

In some embodiments, the first antigen binding domain and the second antigen binding domain of the chimeric antigen receptor comprise the same or different amino acid sequences.

In some embodiments, the first antigen binding domain and the second antigen binding domain of the chimeric antigen receptor are connected directly or indirectly.

In some embodiments, the first antigen binding domain and the second antigen binding domain of the chimeric antigen receptor are connected by a linker.

In some embodiments, the linker comprises an amino acid sequence of (GGGGS)n, wherein the n is any positive integer from 1 to 10.

In some embodiments, the linker comprises an amino acid sequence of (EAAAK)n, wherein the n is any positive integer from 1 to 10.

In some embodiments, the chimeric antigen receptor comprises a costimulatory signal region, wherein the costimulatory signal region comprises an intracellular costimulatory signal region derived from one or more proteins selected from a group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88.

In some embodiments, the costimulatory signal region of the chimeric antigen receptor is an intracellular costimulatory signal region derived from 4-1BB.

In some embodiments, the costimulatory signal region of the chimeric antigen receptor comprises an amino acid sequence as set forth in SEQ ID NO: 4.

In some embodiments, the chimeric antigen receptor comprises an intracellular signaling domain, the intracellular signaling domain comprises an intracellular signal region derived from one or more proteins selected from a group consisting of: CD3ζ, CD3δ, CD3γ, CD3ε, CD79a, CD79b, FcεRIγ, FcεRIβ, FcγRIIa, bovine leukemia virus gp30, Epstein-Barr virus (EBV) LMP2A, simian immunodeficiency virus PBj 14 Nef, Kaposi's sarcoma herpes virus (HSKV), DAP10, DAP-12 and a domain at least containing one ITAM.

In some embodiments, the intracellular signaling domain of the chimeric antigen receptor is a signaling domain derived from CD3ζ.

In some embodiments, the intracellular signaling domain of the chimeric antigen receptor comprises an amino acid sequence as set forth in SEQ ID NO: 5.

In some embodiments, the chimeric antigen receptor comprises a transmembrane region, the transmembrane region comprises a transmembrane domain derived from one or more proteins selected from a group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

In some embodiments, the transmembrane region of the chimeric antigen receptor is a transmembrane region derived from CD8.

In some embodiments, the transmembrane region of the chimeric antigen receptor comprises an amino acid sequence as set forth in SEQ ID NO: 3.

In some embodiments, the chimeric antigen receptor further comprises hinge region, the hinge region comprises a hinge region derived from one or more proteins selected from a group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

In some embodiments, the hinge region of the chimeric antigen receptor is a hinge region derived from CD8.

In some embodiments, the hinge region of the chimeric antigen receptor comprises an amino acid sequence as set forth in SEQ ID NO: 2.

In some embodiments, the chimeric antigen receptor further comprises a low-density lipoprotein receptor-related protein or a fragment thereof.

In some embodiments, the low-density lipoprotein receptor-related protein or the fragment thereof is located at a C terminal of the intracellular signal region.

In some embodiments, the low-density lipoprotein receptor-related protein or the fragment thereof comprises one or more selected from a group consisting of: low-density lipoprotein receptor-related proteins 1-12 and functional fragments thereof.

In some embodiments, the low-density lipoprotein receptor-related protein or the fragment thereof is low-density lipoprotein receptor-related proteins 5 and/or 6 or the fragments thereof.

In some embodiments, the low-density lipoprotein receptor-related protein or the fragment thereof comprises an amino acid sequence as set forth in SEQ ID NO: 7.

In some embodiments, the chimeric antigen receptor further comprises a signal peptide.

In some embodiments, the signal peptide is derived from a signal peptide of CD8 protein.

In some embodiments, the signal peptide of the chimeric antigen receptor comprises an amino acid sequence as set forth in SEQ ID NO: 1.

In another aspect, the present application further provides one or more isolated nucleic acid molecules, which encode the chimeric antigen receptors.

In some embodiments, the nucleic acid molecule further comprises a promoter.

In some embodiments, the promoter is a constitutive promoter.

In some embodiments, the promoter is an EF1$\alpha$ promoter.

In another aspect, the present application further provides a vector, which comprises the nucleic acid molecule.

In some embodiments, the vector is a viral vector.

In some embodiments, the vector is a lentiviral vector.

In another aspect, the present application further provides a cell, which comprises the chimeric antigen receptor, the nucleic acid molecules, and/or the vector.

In some embodiments, the cell is an immune effector cell.

In some embodiments, the cell comprises T cells, B cells, natural killer cells (NK cells), macrophages, NKT cells, monocytes, dendritic cells, granulocytes, lymphocytes, leukocytes, peripheral blood mononuclear cells, embryonic stem cells, lymphoid progenitor cells and/or pluripotent stem cells.

In some embodiments, the cell is a T cell.

In another aspect, the present application further provides a method for preparing the chimeric antigen receptor, the method comprises culturing the cell under a condition enabling the expression of the chimeric antigen receptor.

In another aspect, the present application further provides a method for preparing a modified immune effector cell, the method comprises introducing the vector into the immune effector cell.

In another aspect, the present application further provides a pharmaceutical composition, the pharmaceutical composition comprises the chimeric antigen receptor, the nucleic acid molecule, the vector, and/or the cell, as well as optionally a pharmaceutically acceptable carrier.

In another aspect, the present application further provides use of the chimeric antigen receptor, the nucleic acid molecule, the vector, the cell, and/or the pharmaceutical composition in the manufacture of a medicament for preventing, treating and/or alleviating a disease and/or a disorder.

In some embodiments, the disease and/or the disorder comprises a disease and/or a disorder associated with the abnormal expression of GPRC5D.

In some embodiments, the disease and/or the disorder associated with the abnormal expression of GPRC5D comprises a tumor.

In some embodiments, the tumor comprises a solid tumor.

In some embodiments, the tumor comprises a non-solid tumor.

In some embodiments, the tumor comprises a non-solid tumor.

In some embodiments, the tumor comprises a hematological tumor and/or lymphoma.

In some embodiments, the tumor comprises myeloma.

In some embodiments, the myeloma comprises refractory/relapse multiple myeloma.

In another aspect, the present application further provides a method for preventing, treating and/or alleviating a disease and/or a disorder, the method comprises administering to a subject in need thereof the cell and/or the pharmaceutical composition.

In some embodiments, the disease and/or the disorder comprise a disease and/or a disorder associated with the abnormal expression of GPRC5D.

In some embodiments, the disease and/or the disorder associated with the abnormal expression of GPRC5D comprise a tumor.

In some embodiments, the tumor comprises a solid tumor.

In some embodiments, the tumor comprises a non-solid tumor.

In some embodiments, the tumor comprises a non-solid tumor.

In some embodiments, the tumor comprises a hematological tumor and/or lymphoma.

In some embodiments, the tumor comprises myeloma.

In some embodiments, the myeloma comprises refractory/relapse multiple myeloma.

Those skilled in the art can easily perceive other aspects and advantages of the present application from the detailed description below. In the following detailed description, only exemplary embodiments of the present application are shown and described. As those skilled in the art will recognize, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

**Brief Description of the Drawings**

The specific features of the invention involved in the present application are shown in the appended claims. The characteristics and advantages of the invention involved in the present application can be better understood by referring to the exemplary embodiments and the accompanying drawings described in detail below. A brief description of the drawings is as follows:

FIG. 1 shows a schematic diagram of the components and connection order of each part of the chimeric antigen receptor described in the present application.

FIG. 2A shows the positive rate of T cells after infection with dual-binding domain GPRC5D double $V_HH$ CAR lentivirus.

FIG. 2B shows the positive rate of T cells after infection with a single $V_HH$ CAR lentivirus.

FIG. 3 shows the *in vitro* amplification curve of a single $V_HH$ CAR-T cell.

FIG. 4A shows the expression of CD 107a in dual-binding domain GPRC5D double $V_HH$ CAR-T cells.

FIG. 4B shows the expression of CD 107a in a single $V_HH$ CAR-T cell.

FIG. 5A shows the specific killing of dual-binding domain GPRC5D double $V_HH$ CAR-T cells.

FIG. 5B shows the specific killing of a single $V_HH$ CAR-T cell.

FIG. 6A shows the cytokine secretion level of dual-binding domain GPRC5D double $V_HH$ CAR-T cells.

FIG. 6B shows the cytokine secretion level of a single $V_HH$ CAR-T cell.

FIG. 7A shows the targeted proliferation multiple of dual-binding domain GPRC5D double $V_HH$ CAR-T cells.

FIG. 7B shows the targeted proliferation multiple of a single $V_HH$ CAR-T cell.

FIG. 8 shows the flow chart of CAR-T cell memory phenotype.

FIG. 9 shows the phenotype of $CD8^+$ CAR-T cells.

FIG. 10 shows the expression of $CD3^+$ CAR-T cell activation markers.

FIG. 11 shows the expression of $CD3^+$ CAR-T cell exhaustion markers.

FIG. 12 shows the confocal imaging of the immunological synapse of CAR-T cells.

FIG. 13 shows the quantitative analysis of MTOC and synaptic distance.

FIG. 14 shows the flow chart of *in vivo* tumor inhibition experiment in NSG mice.

FIGs. 15A-15D show the *in vivo* tumor inhibition effect in NSG mice of subcutaneous tumor model.

FIG. 16 shows the *in vivo* tumor inhibition effect in NSG mice of orthotopic transplantation model.

FIG. 17 shows the detection patterns of antibody affinity.

FIG. 18 shows the flow chart of antibody binding activity.

FIG. 19 shows the flow charts of the competitive binding of $G5V_HH1$ and $G5V_HH2$.

FIG. 20 shows the cross-binding activity of the dual-binding domain $V_HH$-hFc antibody with target antigens of different species.

**Detailed Description of the Embodiments**

The implementation of the present application will be illustrated in the following specific examples, and other advantages and effects of the present application will be easily known by those skilled in the art from the content disclosed in the specification.

Definition of Terms

The term "antibody" used herein comprises an intact antibody and a binding fragment thereof. Generally, the fragment competes with the intact antibody from which it is derived for specifically binding to an antigen. Optionally, the antibody or the binding fragment thereof may chemically bind to other proteins or be expressed as a fusion protein with other proteins. For example, the antibody may be a monoclonal antibody, a chimeric antibody, a humanized antibody, and a fully human antibody. For example, the binding protein of the antibody or the binding fragment thereof may comprise GPRC5D. For example, the antibody or the binding fragment thereof may be specific to GPRC5D.

The term "antigen binding fragment" refers to a portion of an intact antibody and refers to the antigenic determining variable region of an intact antibody. For example, the antigen binding fragment may comprise Fab, Fab', F(ab')2, an Fv fragment and a single-chain Fv fragment, a tandem Fv fragment, VHH, and a bispecific antibody. For example, the antigen binding fragment may be a VHH. For example, the antigen binding fragment may bind to GPRC5D. For example, the antigen binding fragment may be specific to GPRC5D.

In the present application, the term "VHH" or "$V_HH$" may be used interchangably and generally refers to an antibody containing the variable antigen binding domain of a heavy chain antibody. VHH can also be referred to as Nanobody (Nb) and/or a single domain antibody. For example, the VHH may bind to GPRC5D. For example, the VHH may be specific to GPRC5D.

In the present application, the antibody may comprise at least two heavy (H) chains and two light (L) chains which are connected to each other through disulfide bonds. Each heavy chain consists of a heavy chain variable region (VH) and a heavy chain constant region. The term "heavy chain constant region" consists of three domains, CH1, CH2 and CH3. Each light chain consists of a light chain variable region (VL) and a light chain constant region. The term "light chain constant region" consists of one domain CL. VH and VL regions may be further subdivided into hypervariable regions called complementarity determining regions (CDRs) interspersed with more conservative regions called framework regions (FRs). Each of VH and VL consists of three CDRs and four FRs, arranged in the following order from the amino terminus to the carboxyl terminus: FR1, CDR1, FR2, CDR2, FR3, CDR3 and FR4. The variable regions of heavy chain and light chain contain binding domains interacting with antigens. The constant region of an antibody can mediate the binding of immunoglobulin to host tissues or factors.

In the present application, the term "G protein-coupled receptor class C group 5 member D (GPRC5D) protein" is an atypical surface orphan receptor. GPRCD5, like other C5 family receptors, has a short amino terminus and is therefore conformationally very similar to the C4 family. Its expression in normal tissues is restricted to hair follicles, but it is also significantly expressed in the bone marrow of multiple myeloma patients and is highly correlated with plasma cell tumor burden and genetic aberrations. For example, the GPRC5D in the present application may specifically refer to the GPRC5D expressed in the MM patients.

In the present application, the term "chimeric antigen receptor" (CAR) generally refers to a recombinant polypeptide that comprises at least an extracellular domain specifically binding to an antigen or a target, a transmembrane region, and an intracellular domain. For example, a hinge region is comprised between the extracellular domain and the transmembrane region. For example, the chimeric antigen receptor may also comprise a low-density lipoprotein receptor-related protein or a fragment thereof. For example, the chimeric antigen receptor may comprise a signal peptide. The binding of the extracellular domain of the CAR to the target antigen on the surface of the target cell leads to CAR clustering and delivers activating stimuli to CAR-containing cells. The CAR redirects the specificity of immune effector cells, and triggers proliferation, cytokine production, and phagocytosis and/or production of molecules capable of mediating death of target antigen-expressing cells in a major histocompatibility complex (MHC)-independent manner. For example, the extracellular domain can specifically bind to GPRC5D.

In the present application, the term "intracellular domain" refers to an intracellular domain containing any truncated portions that are sufficient to transduce the activation signals. The intracellular domain may comprise an intracellular signal region and/or a costimulatory signal region. The term "intracellular signal region" refers to an intracellular region that can generate signals that promote the immune effector function of CAR-containing cells (e.g., CART cells or CAR-expressing NK cells). For example, the intracellular signal region may comprise the intracellular signal regions of one or more proteins selected from a group consisting of: CD3ζ, CD36, CD3γ, CD3ε, CD79a, CD79b, FcεRIγ, FcεRIβ, FcγRIIa, bovine leukemia virus gp30, Epstein-Barr virus (EBV) LMP2A, simian immunodeficiency virus PBj 14 Nef, Kaposi's sarcoma herpes virus (HSKV), DAP10, DAP-12 and a domain at least containing one ITAM. For example, the intracellular signal region may be a signaling domain derived from CD3ζ. The term "costimulatory signal region" refers to a portion of CAR capable of tranducing effector signals in the intracellular signal region. For example, the costimulatory signal region may comprise an intracellular costimulatory signal region derived from one or more proteins selected from a group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88. For example, the costimulatory signal region may be an intracellular costimulatory signal region derived from 4-1BB.

In the present application, the term "transmembrane region" refers to a domain of a peptide, a polypeptide or a protein capable of spanning the cytoplasmic membrane. These domains can be used to anchor an extracellular domain on the cell membrane. For example, the transmembrane region may comprise a transmembrane domain of one or more proteins selected from a group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM. For example, the transmembrane region may be derived from a transmembrane region of CD8.

In the present application, the term "hinge region" represents a portion connecting the CH1 domain to the CH2 domain in the antibody heavy chain polypeptide, e.g., approximately positions 216 to 230 according to the EU numbering system of Kabat. The hinge region is generally a dimeric molecule consisting of two polypeptides having the same amino acid sequence. The hinge region typically comprises about 25 amino acid residues and is flexible, allowing the antigen binding region to move independently. The hinge region can be subdivided into 3 domains: upper, middle, lower hinge domains. For example, the hinge region may comprise a hinge region derived from one or more proteins selected from a group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT. For example, the hinge region may be derived from a hinge region of CD8.

In the present application, the term "low-density lipoprotein receptor-related protein" refers to a class of cell surface proteins belonging to a kind of endocytic receptors, which are widely distributed in living organisms and exhibit great variability in different tissues. Their main function is to uptake cholesterol into cells for cell proliferation and synthesis of steroid hormones and bile salts. For example, the low-density lipoprotein receptor-related protein may be derived from any vertebrates. For example, the low-density lipoprotein receptor-related protein or the fragment thereof may be located at the C terminal of the intracellular signal region. For example, the low-density lipoprotein receptor-related protein or the fragment thereof may comprise one or more selected from a group consisting of: low-density lipoprotein receptor-related proteins 1-12 and functional fragments thereof. For example, the low-density lipoprotein receptor-related protein or the fragment thereof may be a low-density lipoprotein receptor-related protein 6 or a fragment thereof. For example, the low-density lipoprotein receptor-related protein or the fragment thereof may be a low-density lipoprotein receptor-related protein 5 or a fragment thereof.

In the present application, the term "signal peptide" refers to a leader sequence located at the amino terminal (N-terminal) of a nascent CAR protein, which directs the nascent protein to the endoplasmic reticulum during or after the translation for subsequent surface expression. For example, the signal peptide is derived from a signal peptide of CD8 protein.

In the present application, the term "nucleic acid molecule" comprises DNA molecules and RNA molecules. One nucleic acid molecule may be single-chain or double-chain, but preferably is a double-chain DNA. The term "promoter" generally refers to a DNA sequence that can regulate the expression of a selected DNA sequence operably linked to the promoter, thereby affecting the expression of the selected DNA sequence in the cell. For example, the nucleic acid molecule may encode the antigen binding protein and/or the chimeric antigen receptor. For example, the nucleic acid molecule may comprise a promoter. For example, the promoter may be a constitutive promoter. For example, the promoter may be an EF1α promoter.

In the present application, the term "vector" generally refers to a molecule to which one or more nucleic acid molecules of the present application can be attached. For example, the vector may be a viral vector. For example, the vector may be a lentiviral vector.

In the present application, the term "cell" refers to a cell to which a nucleic acid can be transfected. The term "cell" comprises prokaryotic cells for plasmid propagation and eukaryotic cells for expression of nucleic acids and production of encoded polypeptides. For example, the cell may comprise the chimeric antigen receptor, the nucleic acid molecule and/or the vector. For example, the cell may be immune effector cells. The term "immune effector cell" generally refers to immune cells that participate in immune responses and perform effector functions. For example, the effector functions may comprise clearing foreign antigens or promoting immune effector responses. For example, the immune effector cell may comprise T cells, B cells, natural killer cells (NK cells), macrophages, NKT cells, monocytes, dendritic cells, granulocytes, lymphocytes, leukocytes, peripheral blood mononuclear cells, embryonic stem cells, lymphoid progenitor cells and/or pluripotent stem cells. For example, the immune effector cell may be a T cell.

In the present application, the term "pharmaceutical composition" generally refers to chemical or biological compositions suitable for administration to mammalian subjects. For example, the pharmaceutical composition may comprise the antigen binding protein, the chimeric antigen receptor, the polypeptide, the nucleic acid molecule, the vector and/or the cell, as well as optionally a pharmaceutically acceptable carrier. The pharmaceutical composition may be used for preventing, treating and/or alleviating a disease or a disorder associated with the abnormal expression of GPRC5D. For example, the disease or the disorder associated with the abnormal expression of GPRC5D may comprise a tumor. For example, the tumor comprises a solid tumor and/or a non-solid tumor. For example, the tumor may comprise a hematological tumor and/or lymphoma. For example, the tumor may comprise myeloma.

## Detailed Description of the Invention

In one aspect, the present application provides a chimeric antigen receptor, comprising a first antigen binding domain and a second antigen binding domain, the first antigen binding domain targets G protein-coupled receptor class C group 5 member D (GPRC5D) protein, and the second antigen binding domain targets GPRC5D protein.

In some embodiments, the first antigen binding domain and/or the second antigen binding domain of the chimeric antigen receptor may comprise an antibody or an antigen binding fragment thereof. For example, the antigen binding fragment comprises Fab, Fab', F(ab)$_2$, an Fv fragment, F(ab')$_2$, scFv, di-scFv, VHH and/or dAb. For example, the antigen binding fragment comprises a VHH.

In the present application, the CDRs can be divided by the IMGT division method.

The first antigen binding domain

In the present application, the first antigen binding domain of the chimeric antigen receptor may target GPRC5D protein.

In the present application, the first antigen binding domain of the chimeric antigen receptor may comprise at least one CDR of antibody heavy chain variable region VH, the VH may comprise an amino acid sequence as set forth in any one of SEQ ID NO: 15, SEQ ID NO: 19, SEQ ID NO: 24 and SEQ ID NO: 30.

In the present application, the first antigen binding domain of the chimeric antigen receptor may comprise a HCDR3, and the HCDR3 comprises an amino acid sequence as set forth in SEQ ID NO: 33. For example, the first antigen binding domain of the chimeric antigen receptor comprises a HCDR3, and the HCDR3 comprises an amino acid sequence as set forth in any one of SEQ ID NO: 10, SEQ ID NO: 18, SEQ ID NO: 22 and SEQ ID NO: 27.

In the present application, the first antigen binding domain of the chimeric antigen receptor may comprise a HCDR2, and the HCDR2 may comprise an amino acid sequence as set forth in SEQ ID NO: 34 ($IX_1X_2X_3X_4GX_5T$, wherein $X_1$ is N or T, $X_2$ is S or W, $X_3$ is G or S, $X_4$ is D or G, and $X_5$ is N, S or T). For example, the first antigen binding domain of the chimeric antigen receptor may comprise a HCDR2, and the HCDR2 may comprise an amino acid sequence as set forth in any one of SEQ ID NO: 9, SEQ ID NO: 17, SEQ ID NO: 21 and SEQ ID NO: 26.

In the present application, the first antigen binding domain of the chimeric antigen receptor may comprise a HCDR1, and the HCDR1 may comprise an amino acid sequence as set forth in SEQ ID NO: 35. For example, the first antigen binding domain of the chimeric antigen receptor may comprise a HCDR1, and the HCDR1 may comprise an amino acid sequence as set forth in any one of SEQ ID NO: 8, SEQ ID NO: 16, SEQ ID NO: 20 and SEQ ID NO: 25.

In the present application, the first antigen binding domain of the chimeric antigen receptor may comprise a HCDR1, a HCDR2 and a HCDR3, the HCDR1 may comprise an amino acid sequence as set forth in SEQ ID NO: 35, the HCDR2 may comprise an amino acid sequence as set forth in SEQ ID NO: 34 ($IX_1X_2X_3X_4GX_5T$, wherein $X_1$ is N or T, $X_2$ is S or W, $X_3$ is G or S, $X_4$ is D or G, and $X_5$ is N, S or T), and the HCDR3 may comprise an amino acid sequence as set forth in SEQ ID NO: 33.

In the present application, the first antigen binding domain of the chimeric antigen receptor may comprise a HCDR1, a HCDR2 and a HCDR3, and the HCDR1, HCDR2, HCDR3 comprise any one group of amino acid sequences selected from:

(1) the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10;

(2) the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18;

(3) the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22; and

(4) the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27.

In the present application, the first antigen binding domain of the chimeric antigen receptor may comprise a H-FR1, a C-terminal of the H-FR1 is connected to an N-terminal of the HCDR1 directly or indirectly, and the H-FR1 may comprise an amino acid sequence as set forth in SEQ ID NO: 11.

In the present application, the first antigen binding domain of the chimeric antigen receptor may comprise a H-FR2, the H-FR2 is located between the HCDR1 and the HCDR2, and the H-FR2 may comprise an amino acid sequence as set forth in any one of SEQ ID NO: 12, SEQ ID NO: 28 and SEQ ID NO: 23.

In the present application, the first antigen binding domain of the chimeric antigen receptor may comprise a H-FR3, the H-FR3 is located between the HCDR2 and the HCDR3, and the H-FR3 may comprise an amino acid sequence as set forth in SEQ ID NO: 13 or SEQ ID NO: 29.

In the present application, the first antigen binding domain of the chimeric antigen receptor may comprise a H-FR4, an N-terminal of the H-FR4 is connected to a C-terminal of the HCDR3, and the H-FR4 may comprise an amino acid sequence as set forth in SEQ ID NO: 14.

In the present application, the first antigen binding domain of the chimeric antigen receptor may comprise a VH, and the VH may comprise an amino acid sequence as set forth in any one of SEQ ID NO: 15, SEQ ID NO: 19, SEQ ID NO: 24 and SEQ ID NO: 30.

In the present application, the first antigen binding domain of the chimeric antigen receptor may be a VHH, and the VHH may comprise an amino acid sequence as set forth in any one of SEQ ID NO: 15, SEQ ID NO: 19, SEQ ID NO: 24 and SEQ ID NO: 30.

The second antigen binding domain

In the present application, the second antigen binding domain of the chimeric antigen receptor may target GPRC5D protein.

In the present application, the second antigen binding domain of the chimeric antigen receptor may comprise at least one CDR of antibody heavy chain variable region VH, the VH may comprise an amino acid sequence as set forth in any one of SEQ ID NO: 15, SEQ ID NO: 19, SEQ ID NO: 24 and SEQ ID NO: 30.

In the present application, the second antigen binding domain of the chimeric antigen receptor may comprise a HCDR3, and the HCDR3 comprises an amino acid sequence as set forth in SEQ ID NO: 33. For example, the second antigen binding domain of the chimeric antigen receptor comprises a HCDR3, and the HCDR3 comprises an amino acid sequence as set forth in any one of SEQ ID NO: 10, SEQ ID NO: 18, SEQ ID NO: 22 and SEQ ID NO: 27.

In the present application, the second antigen binding domain of the chimeric antigen receptor may comprise a HCDR2, and the HCDR2 may comprise an amino acid sequence as set forth in SEQ ID NO: 34 ($IX_1X_2X_3X_4GX_5T$, wherein $X_1$ is N or T, $X_2$ is S or W, $X_3$ is G or S, $X_4$ is D or G, and Xs is N, S or T). For example, the second antigen binding domain of the chimeric antigen receptor may comprise a HCDR2, and the HCDR2 may comprise an amino acid sequence as set forth in any one of SEQ ID NO: 9, SEQ ID NO: 17, SEQ ID NO: 21 and SEQ ID NO: 26.

In the present application, the second antigen binding domain of the chimeric antigen receptor may comprise a HCDR1, and the HCDR1 may comprise an amino acid sequence as set forth in SEQ ID NO: 35. For example, the second antigen binding domain of the chimeric antigen receptor may comprise a HCDR1, and the HCDR1 may comprise an amino acid sequence as set forth in any one of SEQ ID NO: 8, SEQ ID NO: 16, SEQ ID NO: 20 and SEQ ID NO: 25.

In the present application, the second antigen binding domain of the chimeric antigen receptor may comprise a HCDR1, a HCDR2 and a HCDR3, the HCDR1 may comprise an amino acid sequence as set forth in SEQ ID NO: 35, the HCDR2 may comprise an amino acid sequence as set forth in SEQ ID NO: 34 ($IX_1X_2X_3X_4GX_5T$, wherein $X_1$ is N or T, $X_2$ is S or W, $X_3$ is G or S, $X_4$ is D or G, $X_5$ is N, S or T), and the HCDR3 may comprise an amino acid sequence as set forth in SEQ ID NO: 33.

In the present application, the second antigen binding domain of the chimeric antigen receptor may comprise a HCDR1, a HCDR2 and a HCDR3, and the HCDR1, HCDR2, HCDR3 comprise any one group of amino acid sequences selected from:

(5) the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10;

(6) the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18;

(7) the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22; and

(8) the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27.

In the present application, the second antigen binding domain of the chimeric antigen receptor may comprise a H-FR1, a C-terminal of the H-FR1 is connected to an N-terminal of the HCDR1 directly or indirectly, and the H-FR1 may comprise an amino acid sequence as set forth in SEQ ID NO: 11.

In the present application, the second antigen binding domain of the chimeric antigen receptor may comprise a H-FR2, the H-FR2 is located between the HCDR1 and the HCDR2, and the H-FR2 may comprise an amino acid sequence as set forth in any one of SEQ ID NO: 12, SEQ ID NO: 28 and SEQ ID NO: 23.

In the present application, the second antigen binding domain of the chimeric antigen receptor may comprise a H-FR3, the H-FR3 is located between the HCDR2 and the HCDR3, and the H-FR3 may comprise an amino acid sequence as set forth in SEQ ID NO: 13 or SEQ ID NO: 29.

In the present application, the second antigen binding domain of the chimeric antigen receptor may comprise a H-FR4, an N-terminal of the H-FR4 is connected to a C-terminal of the HCDR3, and the H-FR4 may comprise an amino acid sequence as set forth in SEQ ID NO: 14.

In the present application, the second antigen binding domain of the chimeric antigen receptor may comprise a VH, and the VH may comprise an amino acid sequence as set forth in any one of SEQ ID NO: 15, SEQ ID NO: 19, SEQ ID NO: 24 and SEQ ID NO: 30.

In the present application, the second antigen binding domain of the chimeric antigen receptor may be a VHH, and the VHH may comprise an amino acid sequence as set forth in any one of SEQ ID NO: 15, SEQ ID NO: 19, SEQ ID NO: 24 and SEQ ID NO: 30.

Chimeric antigen receptor

In the present application, the first antigen binding domain and the second antigen binding domain of the chimeric antigen receptor may comprise a HCDR1, a HCDR2 and a HCDR3, respectively.

For example, in the first antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10; in the second antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10.

For example, in the first antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10; in the second antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18.

For example, in the first antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10; in the second antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22.

For example, in the first antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10; in the second antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27.

For example, in the first antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18; in the second antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10.

For example, in the first antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18; in the second antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18.

For example, in the first antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18; in the second antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22.

For example, in the first antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18; in the second antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27.

For example, in the first antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22; in the second antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10.

For example, in the first antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22; in the second antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18.

For example, in the first antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22; in the second antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22.

For example, in the first antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22; in the second antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27.

For example, in the first antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27; in the second antigen binding domain: the HCDR1 comprises an amino acid sequence as set forth in SEQ ID NO: 8, the HCDR2 comprises an amino acid sequence as set forth in SEQ ID NO: 9, and the HCDR3 comprises an amino acid sequence as set forth in SEQ ID NO: 10.

For example, in the first antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27; in the second antigen binding domain: the HCDR1 comprises an amino acid sequence as set forth in SEQ ID NO: 16, the HCDR2 comprises an amino acid sequence as set forth in SEQ ID NO: 17, and the HCDR3 comprises an amino acid sequence as set forth in SEQ ID NO: 18.

For example, in the first antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27; in the second antigen binding domain: the HCDR1 comprises an amino acid sequence as set forth in SEQ ID NO: 20, the HCDR2 comprises an amino acid sequence as set forth in SEQ ID NO: 21, and the HCDR3 comprises an amino acid sequence as set forth in SEQ ID NO: 22.

For example, in the first antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27; in the second antigen binding domain: the HCDR1 comprises an amino acid sequence as set forth in SEQ ID NO: 25, the HCDR2 comprises an amino acid sequence as set forth in SEQ ID NO: 26, and the HCDR3 comprises an amino acid sequence as set forth in SEQ ID NO: 27.

In the present application, the first antigen binding domain and the second antigen binding domain of the chimeric antigen receptor may target the same epitope of GPRC5D. In the present application, the first antigen binding domain and the second antigen binding domain of the chimeric antigen receptor may target different epitopes of GPRC5D.

In the present application, the first antigen binding domain and the second antigen binding domain of the chimeric antigen receptor may comprise the same or different amino acid sequences.

In the present application, the first antigen binding domain and the second antigen binding domain of the chimeric antigen receptor may be connected directly or indirectly. For example, the first antigen binding domain and the second antigen binding domain of the chimeric antigen receptor may be connected by a linker.

In the present application, the linker may comprise an amino acid sequence of (GGGGS)n, wherein the n is any positive integer from 1 to 10. For example, the n may be 1, 2, 3, 4, 5, 6, 7, 8, 9 or 10.

In the present application, the linker may comprise an amino acid sequence of (EAAAK)n, wherein the n is any positive integer from 1 to 10. For example, the n may be 1, 2, 3, 4, 5, 6, 7, 8, 9 or 10.

In the present application, the chimeric antigen receptor may comprise a costimulatory signal region, wherein the costimulatory signal region comprises an intracellular costimulatory signal region derived from one or more proteins selected from a group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88. For example, the costimulatory signal region of the chimeric antigen receptor may be an intracellular costimulatory signal region derived from 4-1BB. For example, the costimulatory signal region of the chimeric antigen receptor may comprise an amino acid sequence as set forth in SEQ ID NO: 4.

In the present application, the chimeric antigen receptor may comprise an intracellular signaling domain, and the intracellular signaling domain comprises an intracellular signal region derived from one or more proteins selected from a group consisting of: CD3ζ, CD3δ, CD3γ, CD3ε, CD79a, CD79b, FcεRIγ, FcεRIβ, FcγRIIa, bovine leukemia virus gp30, Epstein-Barr virus (EBV) LMP2A, simian immunodeficiency virus PBj 14 Nef, Kaposi's sarcoma herpes virus (HSKV), DAP10, DAP-12 and a domain at least containing one ITAM. For example, the intracellular signaling domain of the chimeric antigen receptor may be a signaling domain derived from CD3ζ. For example, the intracellular signaling domain of the chimeric antigen receptor may comprise an amino acid sequence as set forth in SEQ ID NO: 5.

In the present application, the chimeric antigen receptor may comprise a transmembrane region, and the transmembrane region comprises a transmembrane domain derived from one or more proteins selected from a group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM. For example, the transmembrane region of the chimeric antigen receptor may be a transmembrane region derived from CD8. For example, the transmembrane region of the chimeric antigen receptor may comprise an amino acid sequence as set forth in SEQ ID NO: 3.

In the present application, the chimeric antigen receptor may further comprise a hinge region, and the hinge region comprises a hinge region derived from one or more proteins selected from a group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT. For example, the hinge region of the chimeric antigen receptor may be a hinge region derived from CD8. For example, the hinge region of the chimeric antigen receptor comprises an amino acid sequence as set forth in SEQ ID NO: 2.

In the present application, the chimeric antigen receptor may further comprise a low-density lipoprotein receptor-related protein or a fragment thereof. For example, the low-density lipoprotein receptor-related protein or the fragment thereof is located at a C terminal of the intracellular signal region. For example, the low-density lipoprotein receptor-related protein or the fragment thereof comprises one or more selected from a group consisting of: low-density lipoprotein receptor-related proteins 1-12 and functional fragments thereof. For example, the low-density lipoprotein receptor-related protein or the fragment thereof is low-density lipoprotein receptor-related proteins 5 and/or 6 or fragments thereof. For example, the low-density lipoprotein receptor-related protein or the fragment thereof comprises an amino acid sequence as set forth in SEQ ID NO: 7.

In the present application, the chimeric antigen receptor may further comprise a signal peptide. For example, the signal peptide is derived from a signal peptide of CD8 protein. For example, the signal peptide of the chimeric antigen receptor comprises an amino acid sequence as set forth in SEQ ID NO: 1.

In the present application, the low-density lipoprotein receptor-related protein or the fragment thereof in the chimeric antigen receptor can be connected to the C-terminal of CAR through a self-cleaving peptide (e.g., 2A peptides such as T2A, P2A, E2A, etc.). For example, the low-density lipoprotein receptor-related protein or the fragment thereof can be connected to the C-terminal of the intracellular signal region through T2A.

In the present application, from the N-terminal to the C-terminal, the chimeric antigen receptor may sequentially comprise an antigen binding domain binding to the GPRC5D protein, the hinge region, the transmembrane domain, the costimulatory signal region, and the intracellular signal region. For example, from the N-terminal to the C-terminal, the chimeric antigen receptor may sequentially comprise the antigen binding domain, the hinge region derived from CD8, the transmembrane region derived from CD8, the costimulatory signal region derived from 4-1BB, and the intracellular signal region derived from CD3ζ.

In the present application, from the N-terminal to the C-terminal, the chimeric antigen receptor may sequentially comprise an antigen binding domain binding to the GPRC5D protein, the hinge region, the transmembrane domain, the costimulatory signal region, the intracellular signal region, and the low-density lipoprotein receptor-related protein or the fragment thereof. For example, from the N-terminal to the C-terminal, the chimeric antigen receptor may sequentially comprise the antigen binding domain, the hinge region derived from CD8, the transmembrane region derived from CD8, the costimulatory signal region derived from 4-1BB, the intracellular signal region derived from CD3ζ, and the low-density lipoprotein receptor-related protein or the fragment thereof.

For example, the chimeric antigen receptor may comprise an amino acid sequence as set forth in SEQ ID NO: 31 or SEQ ID NO: 32.

In the present application, the chimeric antigen receptor may comprise structures as set forth in the table below:

| CAR | Signal peptide | Extracellular antigen binding domain | | | Hinge region | Transmembrane region | Intracellular signalling | | Transmembrane signal region seq ID |
| --- | --- | --- | --- | --- | --- | --- | --- | --- | --- |
| | | V$_H$H1 | Linker seq ID | V$_H$H2 | | | Costimulatory signal | Primary signal | |
| oriCAR - DG501M | CD8 α | 3B5 V$_H$H | A | 3B5 V$_H$H | CD8 α | CD8 α | 4-1 BB | CD3 ζ | ori |
| oriCAR - DG502M | CD8 α | 385 V$_H$H | A | 3E7 V$_H$H | CD8 α | CD8 α | 4-1 BB | CD3 ζ | ori |
| oriCAR - DG503M | CD8 α | 3B5 V$_H$H | A | 4A2 V$_H$H | CD8 α | CD8 α | 4-1 BB | CD3 ζ | ori |
| oriCAR - DG504M | CD8 α | 3B5 V$_H$H | A | 483 V$_H$H | CD8 α | CD8 α | 4-1 BB | CD3 ζ | ori |
| oriCAR - DG505M | CD8 α | 3E7 V$_H$H | A | 3B5 V$_H$H | CD8 α | CD8 α | 4-1 BB | CD3 ζ | ori |
| oriCAR - DG506M | CD8 α | 3E7 V$_H$H | A | 3E7 V$_H$H | CD8 α | CD8 α | 4-1 BB | CD3 Z | ori |
| oriCAR - DG507M | CD8 α | 3E7 V$_H$H | A | 4A2 V$_H$H | CD8 α | CD8 α | 4-1 BB | CD3 ζ | ori |
| oriCAR - DG508M | CD8 α | 3E7 V$_H$H | A | 4B3 V$_H$H | CD8 α | CD8 α | 4-1 BB | CD3 ζ | ori |
| oriCAR - DG509M | CD8 α | 4A2 V$_H$H | A | 3B5 V$_H$H | CD8 α | CD8 α | 4-1 BB | CD3 ζ | ori |
| oriCAR - DG510M | CD8 α | 4A2 V$_H$H | A | 3E7 V$_H$H | CD8 α | CD8 α | 4-1 BB | CD3 ζ | ori |
| oriCAR - DG511M | CD8 α | 4A2 V$_H$H | A | 4A2 V$_H$H | CD8 α | CD8 α | 4-1 BB | CD3 ζ | ori |
| oriCAR - DG512M | CD8 α | 4A2 V$_H$H | A | 4B3 V$_H$H | CD8 α | CD8 α | 4-1 BB | CD3 ζ | ori |
| oriCAR - DG513M | CD8 α | 4B3 V$_H$H | A | 3B5 V$_H$H | CD8 α | CD8 α | 4-1 BB | CD3 ζ | ori |
| oriCAR - DG514M | CD8 α | 4B3 V$_H$H | A | 3E7 V$_H$H | CD8 α | CD8 α | 4-1 BB | CD3 ζ | ori |
| oriCAR - DG515M | CD8 α | 4B3 V$_H$H | A | 4A2 V$_H$H | CD8 α | CD8 α | 4-1 BB | CD3 ζ | ori |
| oriCAR - DG516M | CD8 α | 4B3 V$_H$H | A | 4B3 V$_H$H | CD8 α | CD8 α | 4-1 BB | CD3 ζ | ori |
| oriCAR - DG517M | CD8 α | 3B5 V$_H$H | B | 3B5 V$_H$H | CD8 α | CD8 α | 4-1 BB | CD3 ζ | ori |
| oriCAR - DG518M | CD8 α | 3B5 V$_H$H | B | 3E7 V$_H$H | CD8 α | CD8 α | 4-1 BB | CD3 ζ | ori |
| oriCAR - DG519M | CD8 α | 3B5 V$_H$H | B | 4A2 V$_H$H | CD8 α | CD8 α | 4-1 BB | CD3 ζ | ori |
| oriCAR - DG520M | CD8 α | 3B5 V$_H$H | B | 4B3 V$_H$H | CD8 α | CD8 α | 4-1 BB | CD3 ζ | ori |
| oriCAR - DG521M | CD8 α | 3E7 V$_H$H | B | 3B5 V$_H$H | CD8 α | CD8 α | 4-1 BB | CD3 ζ | ori |
| oriCAR - DG522M | CD8 α | 3E7 V$_H$H | B | 3E7 V$_H$H | CD8 α | CD8 α | 4-1 BB | CD3 ζ | ori |
| oriCAR - DG523M | CD8 α | 3E7 V$_H$H | B | 4A2 V$_H$H | CD8 α | CD8 α | 4-1 BB | CD3 ζ | ori |
| oriCAR - DG524M | CD8 α | 3E7 V$_H$H | B | 4B3 V$_H$H | CD8 α | CD8 α | 4-1 BB | CD3 ζ | ori |
| oriCAR - DG525M | CD8 α | 4A2 V$_H$H | B | 3B5 V$_H$H | CD8 α | CD8 α | 4-1 BB | CD3 ζ | ori |
| oriCAR - DG526M | CD8 α | 4A2 V$_H$H | B | 3E7 V$_H$H | CD8 α | CD8 α | 4-1 BB | CD3 ζ | ori |

(continued)

| CAR | Signal peptide | Extracellular antigen binding domain | | | Hinge region | Transmembrane region | Intracellular signalling | | Transmembrane signal region seq ID |
|---|---|---|---|---|---|---|---|---|---|
| | | $V_HH1$ | Linker seq ID | $V_HH2$ | | | Costimulatory signal | Primary signal | |
| oriCAR - DG527M | CD8 α | 4A2 $V_HH$ | B | 4A2 $V_HH$ | CD8 α | CD8 α | 4-1 BB | CD3 ζ | ori |
| oriCAR - DG528M | CD8 α | 4A2 $V_HH$ | B | 4B3 $V_HH$ | CD8 α | CD8 α | 4-1 BB | CD3 ζ | ori |
| oriCAR - DG529M | CD8 α | 4B3 $V_HH$ | B | 3B5 $V_HH$ | CD8 α | CD8 α | 4-1 BB | CD3 ζ | ori |
| oriCAR - DG530M | CD8 α | 4B3 $V_HH$ | B | 3E7 $V_HH$ | CD8 α | CD8 α | 4-1 BB | CD3 ζ | ori |
| oriCAR - DG531M | CD8 α | 4B3 $V_HH$ | B | 4A2 $V_HH$ | CD8 α | CD8 α | 4-1 BB | CD3 ζ | ori |
| oriCAR - DG532M | CD8 α | 4B3 $V_HH$ | B | 4B3 $V_HH$ | CD8 α | CD8 α | 4-1 BB | CD3 ζ | ori |

where, A represents (GGGGS)n; B represents B: (EAAAK)n, and n is any positive integer from 1 to 10.

For example, the HCDR1 of 3B5 comprises an amino acid sequence as set forth in SEQ ID NO: 8, the HCDR2 of 3B5 comprises an amino acid sequence as set forth in SEQ ID NO: 9, the HCDR3 of 3B5 comprises an amino acid sequence as set forth in SEQ ID NO: 10, and the VHH of 3B5 comprises an amino acid sequence as set forth in SEQ ID NO: 15.

For example, the HCDR1 of 3E7 comprises an amino acid sequence as set forth in SEQ ID NO: 16, the HCDR2 of 3E7 comprises an amino acid sequence as set forth in SEQ ID NO: 17, the HCDR3 of 3E7 comprises an amino acid sequence as set forth in SEQ ID NO: 18, and the VHH of 3E7 comprises an amino acid sequence as set forth in SEQ ID NO: 19.

For example, the HCDR1 of 4A2 comprises an amino acid sequence as set forth in SEQ ID NO: 20, the HCDR2 of 4A2 comprises an amino acid sequence as set forth in SEQ ID NO: 21, the HCDR3 of 4A2 comprises an amino acid sequence as set forth in SEQ ID NO: 22, and the VHH of 4A2 comprises an amino acid sequence as set forth in SEQ ID NO: 24.

For example, the HCDR1 of 4B3 comprises an amino acid sequence as set forth in SEQ ID NO: 25, the HCDR2 of 4B3 comprises an amino acid sequence as set forth in SEQ ID NO: 26, the HCDR3 of 4B3 comprises an amino acid sequence as set forth in SEQ ID NO: 27, and the VHH of 4B3 comprises an amino acid sequence as set forth in SEQ ID NO: 30.

For example, the CD8α signal peptide may comprise an amino acid sequence as set forth in SEQ ID NO: 1.

For example, the CD8α hinge region may comprise an amino acid sequence as set forth in SEQ ID NO: 2.

For example, the CD8α transmembrane region may comprise an amino acid sequence as set forth in SEQ ID NO: 3.

For example, the 4-1BB costimulatory signal may comprise an amino acid sequence as set forth in SEQ ID NO: 4.

For example, the CD3ζ intracellular signal region may comprise an amino acid sequence as set forth in SEQ ID NO: 5.

For example, the Ori may comprise an amino acid sequence as set forth in SEQ ID NO: 7.

Nucleic acid molecules

In another aspect, the present application further provides one or more nucleic acid molecules, which may be nucleotides, deoxynucleotides and/or ribonucleotides of any length and can encode the chimeric antigen receptor.

For example, the nucleic acid molecules may comprise a promoter. For example, the promoter may be a constitutive promoter. For example, the promoter may be an EF1α promoter.

Vector

In another aspect, the present application further provides a vector that may comprise the nucleic acid molecules. The vector can make the genetic elements it carries be expressed in a host cell by transforming, transducing, or transfecting the host cell. For example, the vector may comprise promoters, transcriptons, enhancers, replicons, selection elements, and reporter genes. For example, the vector may comprise ingredients that help its entry into cells. In order for the nucleic acid molecules to be replicated in the vector, the 5' end and 3' end of the nucleic acid molecules may also comprise long terminal repeat sequences.

For example, the vector may be a viral vector. For example, the vector may be a lentiviral vector.

Cell

In another aspect, the present application further provides a cell that may comprise the chimeric antigen receptor, the nucleic acid molecule and/or the vector. The cell may comprise the progeny of a single cell. Due to natural, accidental, or intentional mutations, the progeny may not necessarily be exactly the same as the original parent cells in terms of the morphology of the total DNA complement or the genome.

In some embodiments, the cell may be an immune effector cell. In some embodiments, the cell may comprise T cells, B cells, natural killer cells (NK cells), macrophages, NKT cells, monocytes, dendritic cells, granulocytes, lymphocytes, leukocytes, peripheral blood mononuclear cells, embryonic stem cells, lymphoid progenitor cells and/or pluripotent stem cells. For example, the cell may be a T cell.

Pharmaceutical composition

In another aspect, the present application further provides a pharmaceutical composition that may comprise the chimeric antigen receptor, the nucleic acid molecule, the vector and/or the cell, as well as optionally a pharmaceutically acceptable adjuvant.

In some embodiments, the pharmaceutical composition may also comprise one or more (pharmaceutically effective) carriers, stabilizers, excipients, diluents, solubilizers, surfactants, emulsifiers and/or preservatives, and other suitable preparations. The acceptable ingredients of the composition are preferably non-toxic to the recipient at the dosage and concentration used. The pharmaceutical composition of the present application may comprise liquid, frozen and freeze-dried compositions.

In some embodiments, the pharmaceutically acceptable adjuvant may comprise any and all solvents, dispersion media, coatings, isotonic agents and absorption delaying agents that are compatible with the medication, which are generally safe, non-toxic and neither biologically nor otherwise undesirable.

In some embodiments, the pharmaceutical composition may be administered parenterally, transdermally, intraperitoneally, intra-arterially, intrathecally and/or intranasally or directly injected into tissues. For example, the pharmaceutical composition may be administered to patients or subjects by means of infusion or injection. In some embodiments, the administration of the pharmaceutical composition can be done in different ways, such as intravenously, intraperitoneally, subcutaneously, intramuscularly, locally, or intradermally.

Preparation method

In another aspect, the present application further provides a method for preparing the chimeric antigen receptor. The method may comprise culturing the cell under a condition enabling the expression of the chimeric antigen receptor.

The present application further provides a method for preparing a modified immune effector cell. The method may comprise introducing the vector into the immune cell.

Use

In another aspect, the present application further provides use of the chimeric antigen receptor, the nucleic acid molecule, the vector, the cell and/or the pharmaceutical composition in the manufacture of a medicament for preventing, alleviating and/or treating a tumor.

In another aspect, the present application further provides a method for preventing, alleviating and/or treating a tumor. The method may comprise administering to a subject the chimeric antigen receptor, the nucleic acid molecule, the vector, the cell and/or the pharmaceutical composition.

In another aspect, the present application further provides the chimeric antigen receptor, the nucleic acid molecule, the vector, the cell and/or the pharmaceutical composition, for use in preventing, alleviating and/or treating a tumor.

In the present application, the tumor may comprise a tumor with the abnormal expression of GPRC5D.

In the present application, the tumor may comprise a solid tumor and/or a non-solid tumor.

In the present application, the tumor may comprise a hematological tumor and/or lymphoma.

In the present application, the tumor may comprise myeloma. In the present application, the myeloma may comprise refractory/relapse multiple myeloma.

In the present application, the subject may comprise human or non-human animals.

In another aspect, the present application provides a kit or a drug delivery device that comprises the chimeric antigen receptor, the nucleic acid molecule, the vector, the cell and/or the pharmaceutical composition.

Without intending to be limited by any theory, the following examples are only to illustrate various technical schemes of the present application, and not intended to limit the scope of the present application.

## EXAMPLES

### Example 1. Screening of Anti-GPRC5D Antibody Using Synthetic Nanobody Library NanoOri_1.0

By aligning 296 repeat-free nanobodies in the PDB database (Protein Data Bank) and the structures thereof, a strategy for mutating CDRs was determined, wherein the CDR3 were 14, 17 and 21 amino acids in length. A commercially available nanobody, Caplacizumab, was selected as backbone to produce the nucleotide sequence of Caplacizumab by gene synthesis, which was then cloned into an HP 153 phage vector. Subsequently, the single-stranded DNA of HP153 was extracted, and the CDRs of the nanobody were mutated by Kunkel Mutagenesis to give a double-stranded DNA. The double-stranded DNA was electrotransfected into competent *E. coli* cells SS320 pre-infected with M13KO7 helper phage. After being cultured overnight, the phage supernatant was collected. Finally, a synthetic nanobody library NanoOri_1.0 with a diversity of $1.44 \times 10^{10}$ (Oricell Therapeutics Co., Ltd.) was constructed as seed bank for screening the antibody sequence.

The GPRC5D full-length sequence (cat: HG24447-UT) commercially available from Sino Biological Company was constructed into in-house existing lentiviral vector, which was transduced with lentivirus to produce over-expressed strains of the target cell CHO-GPRC5D and the target cell HEK293-GPRC5D. Alternate pannings of the synthetic nanobody library (Oricell Therapeutics Co. Ltd.) were carried out using the target cells CHO-GPRC5D and HEK293-GPRC5D for totally 4 rounds.

One was selected from the synthetic nanobody library NanoOri_1.0 and re-precipitated by adding PEG/NaCl for use. The target cells CHO-GPRC5D and HEK293-GPRCSD were selected for use in the alternate screenings of the cell panning. 500 μL of phage and 500 μL of 10% FBS/PBS buffer were mixed to a final volume of 1 mL, which was fed into a 2-mL low-adsorption EP tube, and blocked at 4°C by gentle rotation or periodic mixing for 1 hr. The CHO-GPRC5D cells were harvested by centrifugation at 140 g for 10 min, and washed once with 10 mL of PBS. After counting, the number of viable cells reached 95% or more. The CHO-GPRC5D cells were washed three times with 5% FBS/PBS buffer. Then, the cells were resuspended in 1 mL of 5% FBS/PBS buffer to a cell number of $1 \times 10^7$. During this process, the cells were placed on ice. The CHO-GPRC5D cells were centrifuged at 140 g at 4°C for 2 min, resuspended in 1 mL of the blocked phage antibody library, and combined by gentle rotation or periodic mixing at 4°C or room temperature for 2 hr. The supernatant was removed. The harvested cells were resuspended in 1 mL of 5% FBS/PBS buffer, and repeatedly washed twice. After the last resuspension of the cells, they were transferred to a new 2-mL low-adsorption EP tube to avoid the phage non-specifically adsorbed to the EP tube from elution.

The cells were resuspended in 1 mL of 100 nM Gly-HCl (pH 2.2), incubated at room temperature for 10 min, and then centrifuged at 140 g at 4°C for 2 min. The supernatant was transferred to a new EP tube, and 25 μL of 2M Tris neutralization solution was added. About 1 mL of the neutralization solution was further neutralized with about 500 μL of 7.5% FBS/PBS buffer.

The adsorbed cells in triplicate were centrifuged by the same method as that for the CHO-GPRC5D cells. The eluted and neutralized phages were resuspended in the CHO cells, combined by gentle rotation or periodic mixing at 4°C for 30 min, and centrifuged at 140 g at 4°C for 2 min. The supernatant was taken for next adsorption, totally 3 times. The last phage supernatant was used to infect TG1 cells. The same method was used in the second round of cell panning, except that the number of target cells CHO-GPRC5D was $5 \times 10^6$, and the number of washing was 3. The same operation method was used in the third and the fourth rounds of cell panning, except that the target cells were replaced with HEK293-GPRC5D, the number of cells was $1 \times 10^6$, and the number of washing was increased to 10.

After 4 rounds of panning, it wa observed that the screened phage exhibited significant specific enrichment against the target cells. The panned phage antibody clones were identified using a Cell-based mono phage ELISA: A 96-well ELISA plate was coated with CHO-GPRC5D target cells at $3 \times 10^4$ cells/well at 4°C overnight. Then, non-specific binding sites were blocked with 5% skim milk powder. After full washing, the monoclonal phage supernatant (premixed with the skim milk powder) was added into the 96-well plate, and incubated for 2 hr. After full washing, Anti-M13-HRP was added and reacted for 45 min. After full washing, TMB was added for development. After interaction at room temperature for 5-10 min, the reaction was stopped with 2M sulfuric acid, and the OD value of each well was measured at 450 nm.

After panning, 34 strains of phage antibody clones capable of specifically binding to human GPRC5D were obtained by ELISA assay, which were sequenced to give the single-stranded $V_HH$ gene sequence.

As an example, 3B5 $V_HH$ (with the $V_HH$ amino acid sequence as set forth in SEQ ID NO: 15), 3E7 $V_HH$ (with the $V_HH$ amino acid sequence as set forth in SEQ ID NO: 19), 4A2 $V_HH$ (with the $V_HH$ amino acid sequence as set forth in SEQ ID NO: 24), and 4B3 $V_HH$ (with the $V_HH$ amino acid sequence as set forth in SEQ ID NO: 30) antibody sequences were selected for detecting the cell binding activity between the cell surface target antigen (GPRC5D) with the antibody by flow cytometry fluorescence sorting technology (FACS), using the iQue Screener Flow Cytometer (commercially available from IntelliCyt Company) and with PBS containing 0.1% BSA as buffer.

The target cells (i.e., MM1S myeloma cells) were formulated with buffer to a concentration of $1 \times 10^6$ cells/mL, and added into a 96-well sharp-bottom plate (corning 3894) with 30 μL in each well. The antibody to be detected was formulated with buffer to a concentration of 10 μg/mL, and subject to 3-fold dilution to form a gradient of 8 concentrations. The formulated antibodies of different concentrations were mixed well with the plated target cells at 30 μL/well, and incubated in a refrigerator at 4°C for 1 hr. 150 μL of buffer was added into each well, and centrifuged at 300g for 5 min. The supernatant was discarded, and then the cells were loosened up by shaking, and additionally washed once. The fluorescent secondary antibody (ab98593) was formulated with buffer at a ratio of 1:200, added into each well in an amount of 30 μL and mixed with the cells uniformly, and incubated in the refrigerator at 4°C for 30 min. 150 μL of buffer was added to each well, and centrifuged at 300g for 5 min. The supernatant was discarded, and then the cells were loosened up by shaking, and additionally washed once. 35 μL of buffer was added into each well, mixed well, and the mixture was measured by a flow cytometer. The binding activity of the anti-GPRC5D antibody to the cell surface GPRC5D protein is shown in Table 1. Of those, ET150-5scFv is a positive control antibody with the sequence found in the patent application CN107428829A, 3B5$V_HH$ has an amino acid sequence as set forth in SEQ ID NO: 15, 3E7$V_HH$ has an amino acid sequence as set forth in SEQ ID NO: 19, 4A2$V_HH$ has an amino acid sequence as set forth in SEQ ID NO: 24, and 4B3$V_HH$ has an amino acid sequence as set forth in SEQ ID NO: 30. The results show that each anti-GPRC5D $V_HH$ described in the present application can bind to GPRC5D on the cell surface, while the binding ability is comparable to or even higher than that of the positive control ET150-5 scFv.

Table 1. Binding of Anti-GPRC5D Antibodies to GPRC5D Protein on Cell Surface

| GPRC5D Antibody ($EC_{50}$ μg/mL) | Cell lines | | |
|---|---|---|---|
| | CHO-GPRC5D | K562-GPRC5D | MM1S |
| 3B5 $V_HH$ | 0.1035 | 0.2066 | 0.04387 |
| 3E7 $V_HH$ | 0.4259 | 0.5761 | 0.2865 |
| 4A2 $V_HH$ | 0.06866 | 0.1644 | 0.01948 |
| 4B3 $V_HH$ | 0.1805 | 0.2158 | 0.6580 |
| ET150-5 scFv | 0.2490 | 0.4315 | 0.9109 |

**Example 2. Construction of CAR Lentiviral Expression Vector**

To obtain an anti-GPRC5D CAR-T cell product with stronger affinity and better anti-tumor effect, we combined 4 antibody sequences, $3B5V_HH$, $3E7V_HH$, $4A2V_HH$ and $4B3V_H$, which have good target cell killing activity, high targeted proliferation, and low tonic signal level in a functional verification *in vitro,* into a CAR structure by different peptide linkers to construct dual-binding-domain GPRC5D double $V_HH$ CARs. Exemplary dual-binding-domain double $V_HH$ CAR constructs are shown in Table 1. The constructs were prepared by the following steps. Specially, two $V_HH$ sequences were fused by (GGGGS)n or (EAAAK)n (wherein n can be a positive integer). Then, the CAR lentiviral empty core plasmid (constructed by Oricell Therapeutics Co. Ltd., comprising a new Ori element, hereinafter referred to as CAR lentiviral empty vector) were digested with SphI and NotI endonucleases (commercially available from NEB) to produce an 8992bp linearized fragment. After tapping for recovery, the fragment was mixed with the fused double $V_HH$ fragment at a molar ratio of 1:3 for homologous recombination, and then transformed into DH5α competent cells. Single colony was picked for sequencing identification. The linking order in the dual-binding-domain double $V_HH$ CAR structure is shown in detail in Table 2.

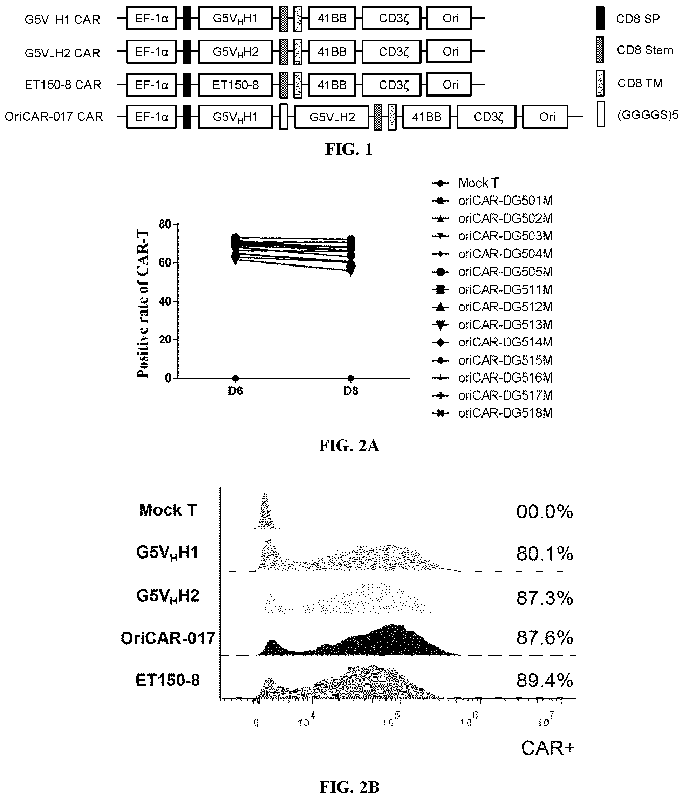

32 dual-binding-domain double $V_HH$ CAR structures in different combination were subject to CAR-T biological functional verifications *in vitro,* comprising *in vitro* proliferation activity, targeted killing ability, cytokine secretion level of the CAR-T cells, and the like. As an example, one double $V_HH$ CAR structure combination was selected and named as OriCAR-017 (i.e., oriCAR-DG508M), and then subject to CAR-T biological functional experiments *in vitro,* with single-domain $G5V_HH1$ CAR (wherein the targeted portion of $G5V_HH1$ has an amino acid sequence as set forth in SEQ ID NO: 19 (3E7) ) and $G5V_HH2$ CAR (wherein the targeted portion of $G5V_HH2$ has an amino acid sequence as set forth in SEQ ID NO: 30 (4B3)), a positive control ET150-8 (with an amino acid sequence found in the Patent No: US10633426B2). Elements in various portions of various CAR lentiviral vectors and the linking order thereof are shown in detail in FIG. 1. Of those, the CD8α signal peptide has an amino acid sequence as set forth in SEQ ID NO: 1, the CD8α hinge region has an amino acid sequence as set forth in SEQ ID NO: 2, the CD8α transmembrane region has an amino acid sequence as set forth in SEQ ID NO: 3, the 4-1BB costimulatory signal has an amino acid sequence as set forth in SEQ ID NO: 4, the CD3ζ intracellular signal transduction region has an amino acid sequence as set forth in SEQ ID NO: 5, and the new Ori element has an amino acid sequence as set forth in SEQ ID NO: 7. An exemplary selection sequence for the linker A is (GGGGS)5, and an exemplary selection sequence for the linker B is (EAAAK)3.

Table 2 Exemplary Dual-Binding-Domain GPRC5D Double V$_H$H CAR Structure Order

| CAR | Signal peptide | Extracellular antigen binding domain | | | Hinge region | Transmembrane region | Intracellular signalling | | Transmembrane signal region seq ID |
|---|---|---|---|---|---|---|---|---|---|
| | | V$_H$H1 | Linker seq ID | V$_H$H2 | | | Costimulatory signal | Primary signal | |
| oriCAR - DG501M | CD8 α | 3B5 V$_H$H | A | 3B5 V$_H$H | CD8 α | CD8 α | 4-1 BB | CD3 ζ | ori |
| oriCAR - DG502M | CD8 α | 3B5 V$_H$H | A | 3E7 V$_H$H | CD8 α | CD8 α | 4-1 BB | CD3 ζ | ori |
| oriCAR - DG503M | CD8 α | 3B5 V$_H$H | A | 4A2 V$_H$H | CD8 α | CD8 α | 4-1 BB | CD3 ζ | ori |
| oriCAR - DG504M | CD8 α | 3B5 V$_H$H | A | 4B3 V$_H$H | CD8 α | CD8 α | 4-1 BB | CD3 ζ | ori |
| oriCAR - DG505M | CD8 α | 3E7 V$_H$H | A | 3B5 V$_H$H | CD8 α | CD8 α | 4-1 BB | CD3 ζ | ori |
| oriCAR - DG506M | CD8 α | 3E7 V$_H$H | A | 3E7 V$_H$H | CD8 α | CD8 α | 4-1 BB | CD3 ζ | ori |
| oriCAR - DG507M | CD8 α | 3E7 V$_H$H | A | 4A2 V$_H$H | CD8 α | CD8 α | 4-1 BB | CD3 ζ | ori |
| oriCAR - DG508M | CD8 α | 3E7 V$_H$H | A | 4B3 V$_H$H | CD8 α | CD8 α | 4-1 BB | CD3 ζ | ori |
| oriCAR - DG509M | CD8 α | 4A2 V$_H$H | A | 3B5 V$_H$H | CD8 α | CD8 α | 4-1 BB | CD3 ζ | ori |
| oriCAR - DG510M | CD8 α | 4A2 V$_H$H | A | 3E7 V$_H$H | CD8 α | CD8 α | 4-1 BB | CD3 ζ | ori |
| oriCAR - DG511M | CD8 α | 4A2 V$_H$H | A | 4A2 V$_H$H | CD8 α | CD8 α | 4-1 BB | CD3 ζ | ori |
| oriCAR - DG512M | CD8 α | 4A2 V$_H$H | A | 4B3 V$_H$H | CD8 α | CD8 α | 4-1 BB | CD3 ζ | ori |
| oriCAR - DG513M | CD8 α | 4B3 V$_H$H | A | 3B5 V$_H$H | CD8 α | CD8 α | 4-1 BB | CD3 ζ | ori |
| oriCAR - DG514M | CD8 α | 483 V$_H$H | A | 3E7 V$_H$H | CD8 α | CD8 α | 4-1 BB | CD3 ζ | ori |
| oriCAR - DG515M | CD8 α | 4B3 V$_H$H | A | 4A2 V$_H$H | CD8 α | CD8 α | 4-1 BB | CD3 ζ | ori |
| oriCAR - DG516M | CD8 α | 4B3 V$_H$H | A | 4B3 V$_H$H | CD8 α | CD8 α | 4-1 BB | CD3 ζ | ori |
| oriCAR - DG517M | CD8 α | 3B5 V$_H$H | B | 3B5 V$_H$H | CD8 α | CD8 α | 4-1 BB | CD3 ζ | ori |
| oriCAR - DG518M | CD8 α | 3B5 V$_H$H | B | 3E7 V$_H$H | CD8 α | CD8 α | 4-1 BB | CD3 ζ | ori |
| oriCAR - DG519M | CD8 α | 3B5 V$_H$H | B | 4A2 V$_H$H | CD8 α | CD8 α | 4-1 BB | CD3 ζ | ori |
| oriCAR - DG520M | CD8 α | 3B5 V$_H$H | B | 4B3 V$_H$H | CD8 α | CD8 α | 4-1 BB | CD3 ζ | ori |
| oriCAR - DG521M | CD8 α | 3E7 V$_H$H | B | 3B5 V$_H$H | CD8 α | CD8 α | 4-1 BB | CD3 ζ | ori |
| oriCAR - DG522M | CD8 α | 3E7 V$_H$H | B | 3E7 V$_H$H | CD8 α | CD8 α | 4-1 BB | CD3 ζ | ori |
| oriCAR - DG523M | CD8 α | 3E7 V$_H$H | B | 4A2 V$_H$H | CD8 α | CD8 α | 4-1 BB | CD3 ζ | ori |
| oriCAR - DG524M | CD8 α | 3E7 V$_H$H | B | 4B3 V$_H$H | CD8 α | CD8 α | 4-1 BB | CD3 ζ | ori |
| oriCAR - DG525M | CD8 α | 4A2 V$_H$H | B | 3B5 V$_H$H | CD8 α | CD8 α | 4-1 BB | CD3 ζ | ori |

| CAR | Signal peptide | Extracellular antigen binding domain | | | Hinge region | Transmembrane region | Intracellular signalling | | Transmembrane signal region seq ID |
|---|---|---|---|---|---|---|---|---|---|
| | | $V_H$H1 | Linker seq ID | $V_H$H2 | | | Costimulatory signal | Primary signal | |
| oriCAR - DG526M | CD8 $\alpha$ | 4A2 $V_H$H | B | 3E7 $V_H$H | CD8 $\alpha$ | CD8 $\alpha$ | 4-1 BB | CD3 $\zeta$ | ori |
| oriCAR - DG527M | CD8 $\alpha$ | 4A2 $V_H$H | B | 4A2 $V_H$H | CD8 $\alpha$ | CD8 $\alpha$ | 4-1 BB | CD3 $\zeta$ | ori |
| oriCAR - DG528M | CD8 $\alpha$ | 4A2 $V_H$H | B | 4B3 $V_H$H | CD8 $\alpha$ | CD8 $\alpha$ | 4-1 BB | CD3 $\zeta$ | ori |
| oriCAR - DG529M | CD8 $\alpha$ | 4B3 $V_H$H | B | 3B5 $V_H$H | CD8 $\alpha$ | CD8 $\alpha$ | 4-1 BB | CD3 $\zeta$ | ori |
| oriCAR - DG530M | CD8 $\alpha$ | 483 $V_H$H | B | 3E7 $V_H$H | CD8 $\alpha$ | CD8 $\alpha$ | 4-1 BB | CD3 $\zeta$ | ori |
| oriCAR - DG531M | CD8 $\alpha$ | 4B3 $V_H$H | B | 4A2 $V_H$H | CD8 $\alpha$ | CD8 $\alpha$ | 4-1 BB | CD3 $\zeta$ | ori |
| oriCAR - DG532M | CD8 $\alpha$ | 483 $V_H$H | B | 4B3 $V_H$H | CD8 $\alpha$ | CD8 $\alpha$ | 4-1 BB | CD3 $\zeta$ | ori |

A: (GGGGS)n, wherein n is an integer greater than or equal to 0, and GGGGS has an amino acid sequence as set forth in SEQ ID NO: 39 B: (EAAAK)n, wherein n is an integer greater than or equal to 0, and EAAAK has an amino acid sequence as set forth in SEQ ID NO: 40

**Example 3. Packaging of Lentivirus and Determination of Virus Titer**

As an example, the lentiviral vector system used to construct the present application belongs to Generation 3, which is comprised of four plasmids, namely, the packaging plasmid pRH1 encoding the Gag-Pol protein, the packaging plasmid pVH3 encoding the Rev protein and the pMH2 plasmid encoding the envelope protein VSV-G, as well as the core plasmid, i.e., various CAR lentiviral plasmids containing $V_HH$ sequence in the above Example 2. The expression of CAR gene in various CAR lentiviral plasmids is regulated by an elongation factor-1$\alpha$ (EF-1$\alpha$) promotor.

3.1 Packaging of Lentivirus:

(1) About $2.5\times10^6$ 293T cells in good growth state were added into 5 mL of complete medium for 293T cells (high-sugar DMEM containing 10% FBS), mixed well, and inoculated in a 6-cm culture dish (ensuring that the confluence of cells was 80-90% after 20-24h), and cultured in an incubator at 37°C, 5% $CO_2$ overnight.

(2) After about 20-24h, three types of plasmids (totally 6.9 $\mu$g, at a mass ratio of pRH1:pMH2:pVH3:CAR lentiviral core plasmid = 1:1:2:2) were added to 300 $\mu$L of Opti-MEM medium and mixed well. 20.7 $\mu$L of FuGENEHD transfection reagent (Promega, E2311) was added, carefully mixed well, and stood at room temperature for 15-20 min. Then, the mixed solution was carefully dropped into a 6-cm culture dish, which was carefully shaked, and cultured in an incubator at 37°C, 5% $CO_2$ overnight.

(3) About 20-24 h after transfection, the supernatant liquor in the 6-cm culture dish was carefully discarded, and then 5 mL of complete medium for 293T cells was slowly added.

(4) About 48 h after transfection, the supernatant liquor (containing dead cells and debris) was collected from the 6-cm culture dish, and centrifuged at 3000 g for 5 min. The supernatant virus solution in which the cell debris was removed was transferred to a new centrifuge tube, sub-packaged, and stored at -80°C.

3.2 Measurement of Lentivirus Titer:

(1) The prepared virus solution was rapidly thawed.

(2) 293T cells in good growth state (typically, cells cultured within 20 passages) were collected, and the supernatant was discarded. The cells were washed with PBS, and digested with 0.25% trypsin (GIBCO) at 37°C for about 3 min. After the cells were fully digested, a volume of complete medium for 293T cells was added to stop the reaction. The culture was sampled for counting, and adjusted to a cell density of $2.0\times10^5$/mL. Polybrene was added to a final concentration of 10 $\mu$g/mL, and then the cells were inoculated into a 6-well plate at an inoculation amount of 2.5 mL per well.

(3) Then, the above virus solutions were added into the 6-well plate, respectively. Typically, each virus solution was added in three loading gradients of 2 $\mu$L, 5 $\mu$L and 10 $\mu$L, and 1-2 blank wells without virus solution were needed as negative control in the detection by flow cytometry.

(4) The cell culture plate was placed in an incubator at 37°C, 5% $CO_2$ for static culture.

(5) After the cells were subject to the static culture for 48 h, the supernatant waste was discarded. The cells were washed with PBS, and digested with 0.25% trypsin (GIBICO) at 37°C for about 3 min. After the cells were fully digested, a volume of complete medium for 293T cells was added to stop the reaction. The culture was sampled for counting, and then $5\times10^5$ cells were transferred to a 1.5 mL sterile centrifuge tube and labeled.

(6) The culture solution was supplemented with an NBS solution (a PBS solution containing 1% fetal bovine serum) to 1.2-1.5 mL, and centrifuged at 4°C at 500 g for 5 min. Then, the supernatant was discarded.

(7) 1 mL NBS was added into each tube, mixed well by gentle beating, and centrifuged at 4°C at 500 g for 5 min. The supernatant was fully discarded.

(8) 50 $\mu$L NBS and 0.8 $\mu$g Biotin-Goat anti-human F(ab)2 antibody (109-066-006) were added to each tube, mixed well by gentle beating with pipette (with no primary antibody added into the negative control), and incubated at 4°C for 60 min.

(9) After the incubation was completed, 1 ml NBS was directly added to resuspend the cells, which were centrifuged at 4°C at 500 g for 5 min. Then, the supernatant was discarded.

(10) 50 $\mu$L NBS and 0.5 $\mu$g APC streptavidin (554067) fluorescent secondary antibody were added into each tube (such protein was not added into the negative control), and incubated at 4°C for 30 min.

(11) The step (9) was repeated.

(12) 200 $\mu$L NBS was added to each tube to resuspend the cells.

(13) The percentage of fluorescence expression was detected by BD FACS CantoII flow cytometer, and the data was processed by FlowJo-V10 software.

Only if the positive rate of each test tube - the positive rate of the negative control is 5%-20%, the result is available. The virus titer is calculated following the formula as below:

$$\text{Titer of Lentivirus } (TU/mL)$$
$$= \frac{(number\ of\ plated\ cells \times (positive\ rate\ of\ test\ tubes - positive\ rate\ of\ control\ tubes))}{volume\ of\ inoculated\ virus\ solution\ (mL)}$$

After detection, the various $V_HH$-containing CARs as above have a virus titer in a range of $1\text{-}10\times10^6$ TU/mL.

**Example 4. Infection of T Cells and In Vitro Amplification of GPRC5D CAR-T Cells**

The dual-binding-domain GPRC5D double $V_HH$ CAR antibody sequences in various combination and the CAR-T cell with an individual $V_HH$ GPRC5D antibody sequence are prepared by the following method.

(1) Human periphery blood mononuclear cells (PBMCs) were isolated from blood samples collected by the apheresis machine through Ficoll density gradient centrifugation (wherein the apheresis components were provided by volunteers of Ark Project of Shanghai Origincell Group).
(2) CD3+ T cells with a purity of >95% were obtained by sorting of the PBMCs by CD3 positive selection magnetic beads, and the method for sorting the T cells are described in detail in the instructions (MACS, DS130-050-101).
(3) The CD3+ T cells were resuspended in a complete medium for T cells (X-VIVO$^{15}$ (Lonza) + 5% FBS + cytokine), and the washed CD3/CD28 Dynabeads (Gibco, 40203D) were added at a 3-fold proportion. Then, additional complete medium for T cells was added to adjust the cell density to $1.0\text{-}1.2\times10^6$ cells/mL. The culture solution was placed in an incubator at 37°C, 5% $CO_2$ for activation culture.
(4) Typically, a lentiviral transduction was carried out after the T cells were activated for 20-24 h.
(5) T cells which had been activated for 20-24 h were collected, centrifuged at 500g for 5 min, resuspended in a volume of complete medium for T cells, and then sampled for counting. At a ratio of the virus multiplicity of infection (MOI) of 3, the virus solutions with titers measured in Example 3 were added, respectively, and then polybrene was added to a final concentration of 5 $\mu$g/mL. Additional complete medium for T cells was added to adjust the cell density to $0.6\text{-}1.0\times10^6$ cells/mL, and the cells were placed in an incubator at 37°C, 5% $CO_2$ for culture. An additional sample of T cells without lentiviral infection was used as the negative control group.
(6) After about 20-24 h of virus transduction, T cells were collected from various groups, centrifuged at 500g for 5 min, resuspended in a volume of complete medium for T cells, and sampled for counting. Additional complete medium for T cells was added to adjust the cell density to $0.5\text{-}0.7\times10^6$ cells/mL.
(7) After then, the CAR-T cells were counted every 1-2 days, and additional complete medium for T cells was added according to the actual conditions to adjust the cell density to $0.5\text{-}1.0\times10^6$ cells/mL.
(8) Generally, Dynabeads were removed on Day 5 of the culture, and the total culture period was about 12 days.

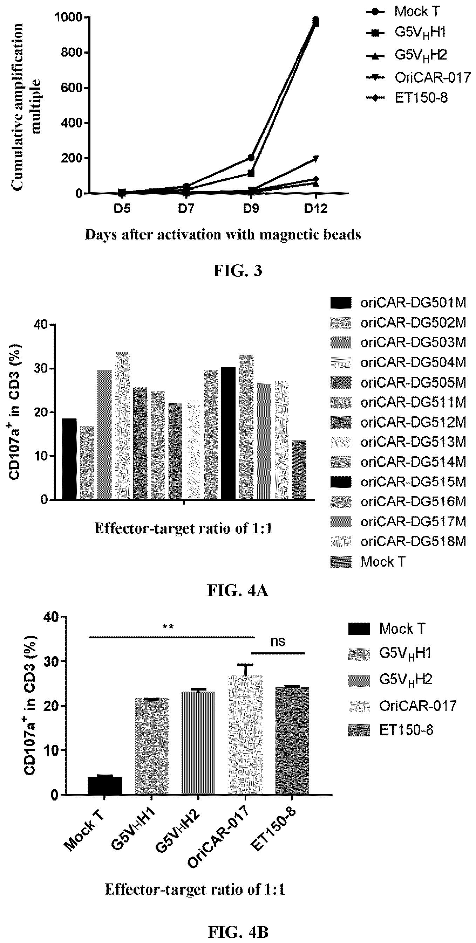

The experimental results are shown in FIGS. 2A-2B. After amplification by activation culture, the T cells infected with the dual-binding-domain GPRC5D double $V_HH$ CAR lentivirus have a CAR positive rate between 55% and 75%; and the individual $V_HH$ GPRC5D CAR has a positive rate between 80% and 90%. The CAR gene is stably expressed on the T cells, and there is no significant difference among the various groups. As shown in FIG. 3, after *in vitro* stimulation by CD3/CD28, the CAR-T cells proliferate stably. In particular, from Day 9 of the co-culture, the G5$V_H$H1 CART and OriCAR-017 cells exhibit superior proliferation potential as compared with G5$V_H$H2 CAR-T and the positive control ET150-8 CAR-T cells with the cumulative amplification multiples of 1000 and 200, respectively; which can be used in the cytological function experiments.

**Example. 5 Determination of Expression Level of Degranulation Molecule CD107a in GPRC5D CAR-T Cells**

One of the important ways for CAR-T cells to exert cytotoxicity is to release perform and granzyme, and the degranulation effect of the CAR-T cells is also an important manifestation of CART cell activation. Therefore, the expression level of cell degranulation functional molecule CD107a is a key index to evaluate the killing effect of CART cells. To detect the activation ability of CAR-T against the target cells, we detected the expression level of CD107a in CD3 positive cells in the co-culture wells, and judged the activation and killing ability of CAR-T by the level.
As shown in FIGS. 4A-4B, after co-culture of different dual-binding-domain GPRC5D double $V_HH$ CAR-T cells

with the target cells at an effector-target ratio of 1:1 for 4 h, the expression level of CD107a in various groups of the CAR-T cells are not uniform, and all higher than the expression level of CD107a in Mock T control cells; wherein the expression positive rate of CD107a in OriCAR-017 is around 28%, which is relatively higher than the expression positive rates of CD107a in the two single-domain antibody CART cell groups and the positive control group of ET150-8. The experiment shows that OriCAR-017 has a significant degranulation effect on specific target cells, indicating good activation and effect killing potential.

**Example 6. Evaluation of In Vitro Effect Killing Activity of GPRC5D CAR-T Cells**

To accurately detect the killing ability of the CAR-T cells against the target cells, we introduced the GPRC5D gene into the CHO cells by means of lentivirus, and then sorted the GPRC5D positive cells, namely, CHO-GPRC5D cells, by a flow sorter. The target cells are the CHO-GPRC5D cells, and the negative cells are the CHO negative cells. The effector cells cultured to Day 9 were incubated with the target cells at an effector-target ratio of 3:1 for 20 h, and the survival of the target cells (i.e., the killing ability of the CAR-T cells) was determined by the LDH method.

The killing toxicity of cells can be calculated following the formula below:

$$\text{Killing rate\%} = \frac{\text{Experimental well} - \text{Spontaneous Release of Target cells} - \text{Spontaneous Release of Effector Cells}}{\text{Maximum Release of Target Cells} - \text{Spontaneous Release of Target Cells}}$$

$$\times \, 100$$

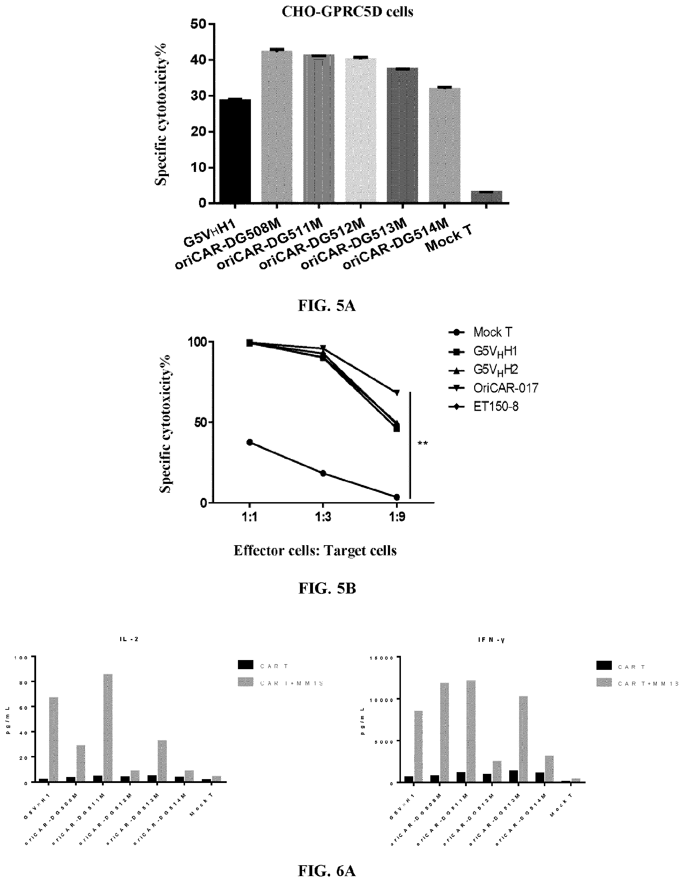

As shown in FIGS. 5A-5B, as compared with the blank effector cells T cells, the results show that the dual-binding-domain GPRC5D double $V_H$H CAR-T cells have significant killing effect against the target cells; and the individual $V_H$H CAR-T cells have significant killing effect against the target cells. Compared with Mock T cells, the single-domain antibody G5$V_H$H1 CAR-T, the single-domain antibody G5$V_H$H2 CAR-T, the dual epitope-targeting OriCAR-017 and the positive control ET150-8 all exhibit significant killing effect against the target cells. In particular, at an effector-target ratio of 1:9, the killing effect of OriCAR-017 against MM.1S target cells is significantly higher than those of the single-domain antibody CAR-T and the positive control ET150-8, achieving 70%.

**Example 7. Cytokine Secretion of GPRC5D CAR-T Cells After In Vitro Killing**

Referring to Example 6, a target cell killing experiment was conducted in a 96-well plate at a ratio of the effector cells to the target cells of 1:1. The target cells and the effector cells were added in order, with the number of the effector cells in each of the 96 wells set as $2 \times 10^4$. Two duplicate wells were set for each effector-target ratio in each group, and two individual effect cell wells were additionally provided for each group to detect the background factor secretion of the effector cells.

Around 24 hours after the killing of the target cells, the 96-well plate was centrifuged, and the supernatant was collected from each well. The effector cells were centrifuged to remove the supernatant, and diluted with X-VIVO to $3 \times 10^5$ cells/mL. 100 $\mu$L was taken from each well for use. Non-target cells (i.e., the CAR-T cells which were not co-cultured with tumor cells) and MM.1S cells were mixed at an effector-target ratio of 1:1 in a round-bottom 96-well plate. After 24 hours of co-culture, the supernatant was pipetted and detected by an R&D Cytokine Kit for levels of IL-2 and IFN-y in the supernatant.

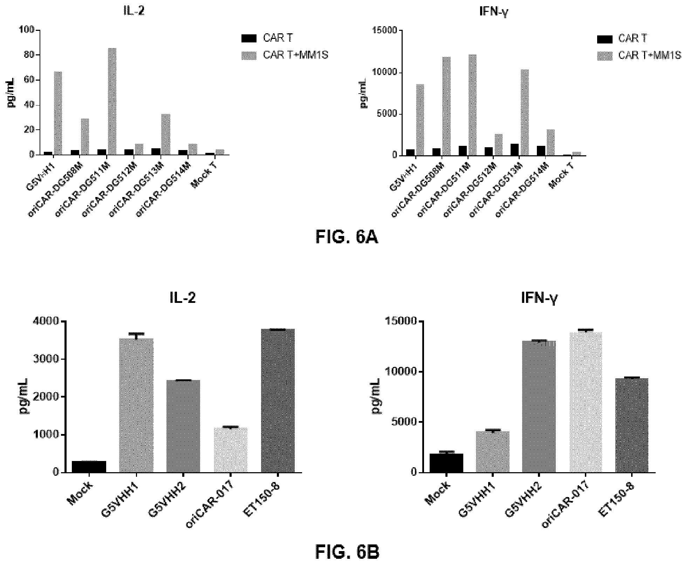

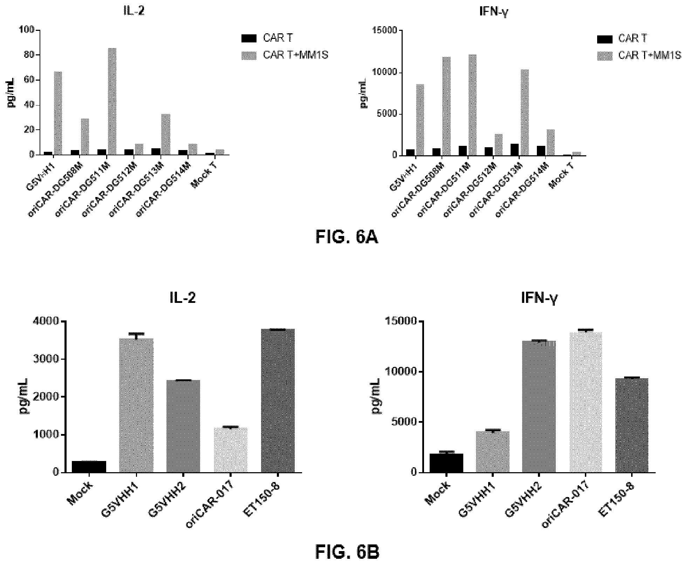

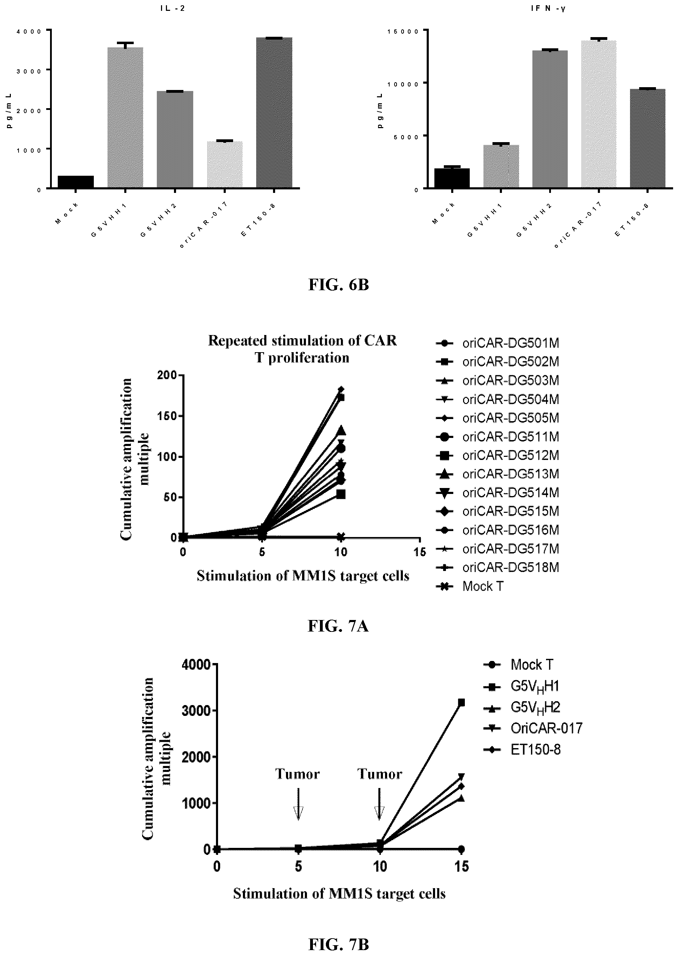

As shown in FIGS. 6A-6B, under the stimulation of MM.1S target cells, the secretion level of both IL-2 and IFN-y in the dual-binding-domain GPRC5D double $V_H$H CAR-T cells are higher than those in the group of Mock T cells; the secretion of IFN-y in various CAR-T groups is significantly higher than that in the group of Mock T cells. As compared with the two single-domain antibody CAR-T cell groups and the positive control group of ET150-8, the dual epitope-targeting OriCAR-017 has higher secretion level of IFN-y, indicating a strong antigen-specific activation effect of the OriCAR-017. In addition, the secretion of IL-2 in various CAR-T groups is significantly higher than that in the group of Mock T cells, but the IL-2 secretion level of the OriCAR-017 is lower than that of the single-domain antibody CAR-T cells, indicating that OriCAR-017 has certain activation and proliferation properties.

**Example 8. Targeted Proliferation Ability of GPRC5D CAR-T Cells**

In this example, untreated MM.1S cells were used as the activation target cells to detect the targeted proliferation ability of the CAR-T cells.

Generally, the detection was conducted around Day 9 to Day 12 during the culture of CAR-T cells. The CAR positive rate of various groups should be previously detected, and adjusted to be consistent therebetween using blank effector T cells (wherein the medium is X-VIVO15 medium without any additives).

The CAR-T cells and MM.1S cells were subject to a first round of targeted stimulation at an effector-target ratio of 1:1. After 5 days of co-incubation, all the cells were collected, and sampled for counting. Then, a second round of targeted stimulation was conducted at a ratio of 1:3 (total CAR-T cells:fresh MM.1S cells). After additional 4-5 days of co-incubation, a third round of targeted stimulation was conducted (wherein the ratio of the two types of cells was the same as that in the second round of targeted stimulation). During the three rounds of targeted stimulation, an appropriate amount of X-VIVO15 medium was supplemented every 1-2 days in accordance with the cell growth.

As shown in FIG. 7A, the dual-binding-domain GPRC5D double $V_HH$ CAR-T cells have different degrees of targeted proliferation ability after being stimulated by the target cells, and the proliferation multiple is between 50 and 200 after two rounds of repeated stimulation, which is significantly different from that of the control Mock T cells, indicating that the dual-binding-domain GPRC5D double $V_HH$ CAR-T cells can be effectively and rapidly amplified against a specific tumor in organisms. As shown in FIGS. 7B, various groups of CAR-T cells exhibit strong cell proliferation ability after being stimulated by the MM.1S target cells, and the proliferation multiple can reach 1,000 to 3,500 after three rounds of target cell stimulation. As compared with the control mock T cells, the proliferation activity is significantly increased, wherein the $G5V_HH1$ CAR T cells exhibit the highest amplification efficiency under the stimulation of target cells. Moreover, the OriCAR-017 cells have higher targeted proliferation amplification efficiency than the positive control ET150-8 CAR T cells. This result shows that the OriCAR-017 CAR T cells stimulates a rapid proliferation in the target cells, indicating that they can be effectively and rapidly amplified against a specific tumor in organisms.

**Example 9. Cell Phenotype Analysis of GPRC5D CAR-T Cells**

After a positive result was obtained in the GPRC5D CAR expression detection of CD3+ T cells activated by lentivirus infection, the cells were tested for their memory phenotype, activation phenotype and depletion phenotype on Day 12 after co-culture: separately collecting four groups of effector CAR T cells (comprising $G5V_HH1$ CAR-T, $G5V_HH2$ CAR-T, OriCAR-017 cells, and the positive control ER150-8) for detecting by flow cytometry: the memory markers in various groups of CD3+ T cells, comprising the proportions of CD45RO, CCR7, and CD62L cells; the activated markers in various groups of CD3+ T cells, comprising the proportions of CD25 and CD69 cells; and the depleted markers in various groups of CD3+ T cells, comprising the proportions of PD-1, LAG-3 and TIM-3 cells.

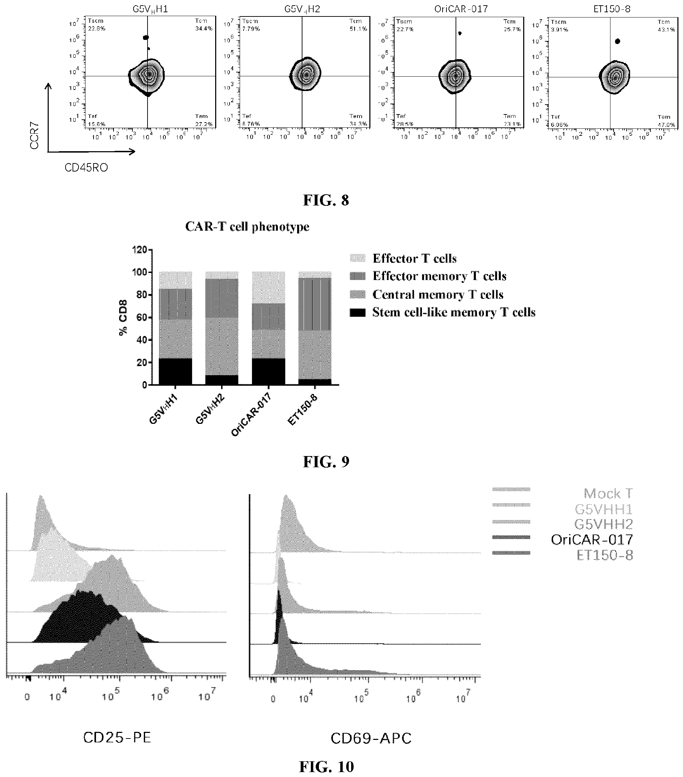

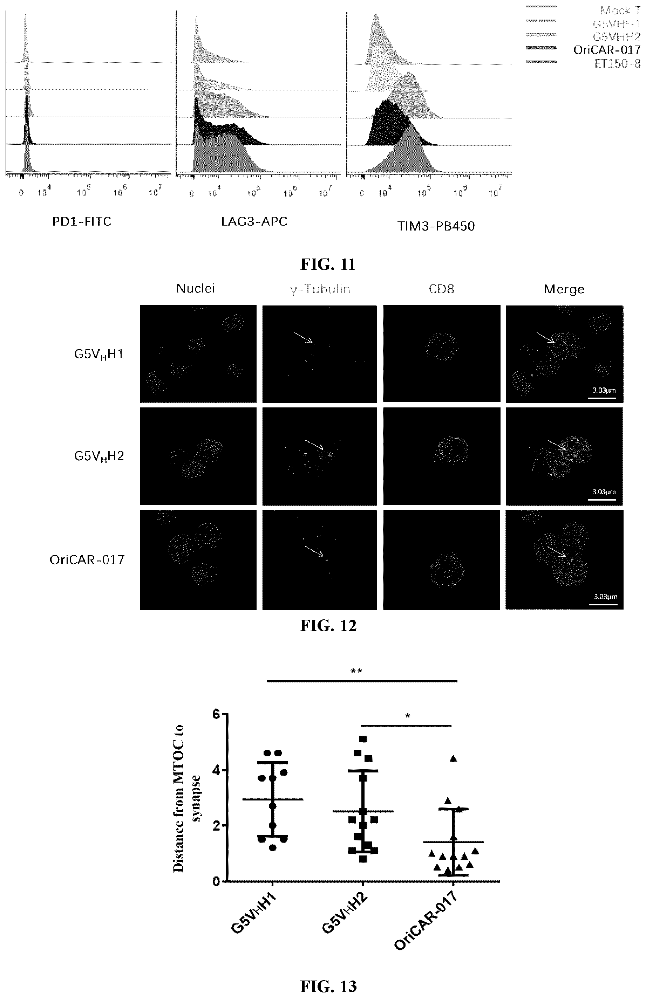

As shown in Table 3 and FIGS. 8 and 9, on Day 12 of the CAR-T cell culture, as compared with $G5V_HH2$ ($T_{SCM}$: 7.79%; $T_{CM}$: 51.1%) and ET150-8 CAR-T cells ($T_{SCM}$: 3.91%; $T_{CM}$: 43.1%), OriCAR-017 ($T_{SCM}$: 22.7%; $T_{CM}$: 25.7%) and $G5V_HH1$ CAR-T cells ($T_{SCM}$: 22.8%; $T_{CM}$: 34.4%) exhibit higher stem cell memory ($T_{SCM}$) phenotype and low central memory T cell ($T_{CM}$) phenotype; and the T cells have low differentiation degree. As shown in FIG. 10, on Day 12 of the CAR-T cell culture, OriCAR-017 and $G5V_HH1$ CAR T cells have lower expression level of activated markers CD25 and CD69 that those of $G5V_HH2$ and ET150-8 CAR T cells, that is, T cells have low activation degree, and OriCAR-017 T cells have an advantage of low self-activation cell phenotype. As shown in FIG. 11, on Day 12 of the CAR-T cell culture, OriCAR-017 T cells have lower expression level of depleted markers LAG-3 and TIM-3 than those of $G5V_HH2$ and ET150-8 CAR-T cells, that is, T cells have low depletion degree, and OriCAR-017 T cells have an advantage of low depletion cell phenotype.

Table 3. Proportion of Memory T cell Subpopulation

|  | $T_{SCM}$ % | $T_{CM}$ % | $T_{EM}$ % | $T_{EF}$ % |
|---|---|---|---|---|
| $G5V_HH1$ | 22.8 | 34.4 | 27.2 | 15.6 |
| $G5V_HH2$ | 7.79 | 51.1 | 34.3 | 6.76 |
| OriCAR-017 | 22.7 | 25.7 | 23.1 | 28.5 |
| ET150-8 | 3.91 | 43.1 | 47 | 6.06 |

**Example 10. Study on Immunosynaptic Mechanism of GPRC5D CAR-T Cells**

Immune synapse (IS) is a structure formed by linking to a specific activation receptor, which is a key step for the antigen recognition, proliferation, and activation of CAR-T cells, and an important component of cellular immune response and humoral immune response. The formation of immune synapses is related to the effector function of CAR-T cells. At the CAR-T level, the polarization of microtubule organizing center (MTOC) of immune synapse structure was measured to further evaluate the functional advantages of the CAR-T cells. The CAR-T cells and the target cells were co-cultured at a ratio of 1:1 for 5 min, then transferred to Nunc chamber slides, incubated for 20 min, fixed in 4% PFA at room temperature, and washed with 3×PBS. The cells were incubated with an immunostaining permeabilization buffer (Triton X-100) at room temperature; incubated with a 10% equine serum/immunostaining permeabilization buffer (Triton X-100) at room temperature; incubated with primary antibody diluted with 2% equine serum/immunostaining permeabilization buffer (Triton X-100) at room temperature; and incubated with secondary antibody diluted with 2% equine serum/immunostaining permeabilization buffer (Triton X-100) at room temperature. The slides were separated, sealed with ProLong Diamond, dried for solidification, and then detected by 100-fold laser co-focal microscope with oil lens under conditions of: ultraviolet, 488 nm and 543 nm, 1024×1024, Z-axis scanning at 0.4 $\mu$m to give 3D images and videos by superposition.

As shown in FIG. 12, the G5$V_H$H1, G5$V_H$H2 and OriCAR-017 CAR T cells were co-focally imaged; wherein the cell nuclei were stained to blue, the $\gamma$-tubulin was stained to green, the T cells were stained to red, the white arrows represented the polarization positions of the microtubule organizing center (MTOC) at IS. The closer the distance between MTOC and IS is, the more stable the IS structure is. As shown in FIG. 13, compared with the G5$V_H$H1 and G5$V_H$H2 CAR-T cells, the polarization level of MTOC to IS in the OriCAR-017 cells is significantly increased, which is manifested in that the distance between MTOC and IS is reduced to give a superior, more stable IS structure, thereby having superior functional advantages, indicating that the cytotoxicity mediated by the OriCAR-017 cells is better than those of the mono-targeting GPRC5D $V_H$H1 and GPRC5D $V_H$H2 CAR-T cells.

## Example 11. In Vivo Experiments of GPRC5D CAR-T Cells in Mice

We further demonstrated the tumor-eliminating and tumor-suppressing effect of the OriCAR-017 CAR-T cells in NSG mice. Specifically, the experiment was divided into 5 groups: Mock T cells, G5$V_H$H1 CAR-T, G5$V_H$H2 CAR-T, ET150-8 CAR-T and OriCAR-017 CAR-T cell groups, with 5 mice in each group. First, the NSG mice were subcutaneously inoculated with MM1S cells at a dose of $1\times10^7$ cells per mouse. About 2 weeks later (with tumor size of 50-150 mm$^3$), the CAR-T (or T) cells that have been cultured for 10-12 days were injected into the tail vein at a dose of $1.6\times10^6$ CAR-T cells per mouse (adjusting with T cells to allow the total inoculating cell number in each mouse to be consistent). After the inoculation of CAR-T (or T) cells, the periphery blood of mice was detected for the cytokine secretion on Day 7 and Day 14 after the inoculation of the CAR-T (or T) cells, and the tumor size and weight of mice were measured 3 times every week for totally 3 weeks.

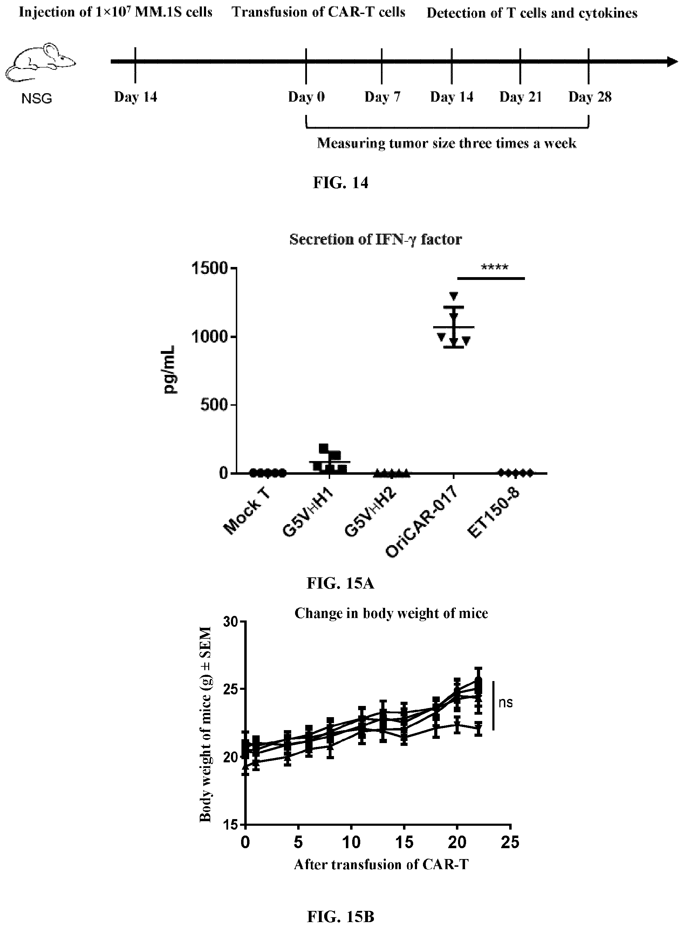

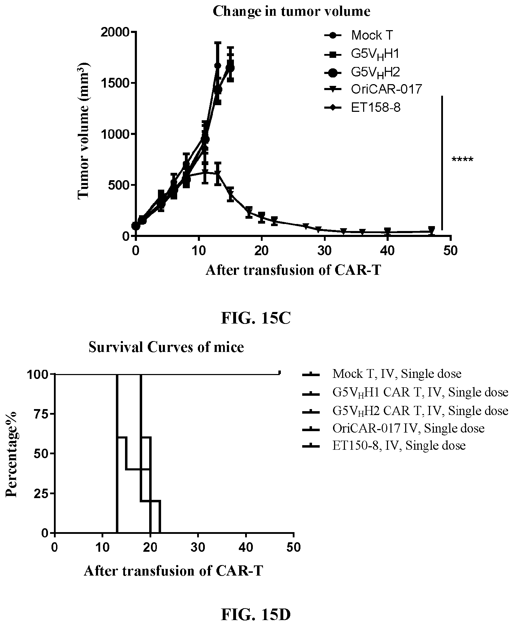

The procedure of *in vivo* tumor-suppressing experiment in NSG mice is shown in FIG. 14. As shown in FIG. 15A, the secreted cytokine IFN-y was detected on Day 7 after injection. The IFN-y produced by the OriCAR-017 CAR-T cell group is significantly higher than those of G5$V_H$H1 CAR-T, G5$V_H$H2 CAR-T, and the positive control ET150-8 CAR-T. As for the safety, as shown in FIG. 15B, the weight of mice presents a stably increasing tendency over time, and the mice in all the CAR-T cell experiment groups and the Mock T group have consistent tendency of weight curves. Moreover, after the injection of OriCAR-017, the mice had glossy hairs, and normal behaviors and vital signs, showing that the OriCAR-017 has good safety in mice. As for the *in vivo* tumor-suppressing effect, as shown in FIG. 15C, compared with the single-binding-domain G5$V_H$H1 CAR-T and G5$V_H$H2 CAR-T cells and the positive control ET150-8 CAR-T cells, the OriCAR-017 cell group exhibits highly significant anti-tumor effect with the tumor-suppressing rate approaching 100%. As shown in FIG. 15D, the mice in the OriCAR-017 cell group achieve 100% survival during the study. The survival rate of mice in this group is much higher than those of the single-binding-domain $V_H$H CAR T cell group and the Positive control group of ET150-8 CAR-T cells, indicating that the OriCAR-017 may exhibit good anti-tumor activity clinically.

To further simulate the distribution of blood tumor in human body, we further demonstrated the tumor-eliminating and tumor-suppressing effect of the OriCAR-017 cells in the B-NSG mice based on living image technology. The B-NSG mice were injected with MM1S-Luciferase target cells in their tail vein, and randomly grouped according to the bioluminescence intensity of mice, with 5 mice in each group. The mice were injected with different doses of pre-cryopreserved OriCAR-017 CAR-T or Mock T cells in their tail vein. After the refusion of CAR-T, the mice were observed with living animal imaging system on each Tuesday and Thursday for their change in bioluminescence in the mice and for their weight and health conditions. During this procedure, the mice underwent blood sampling from eyeball or tail vein three times for the detection of cytokine, and the proportion of CD3$^+$CD8$^+$T cells was detected by BD flow cytometry.

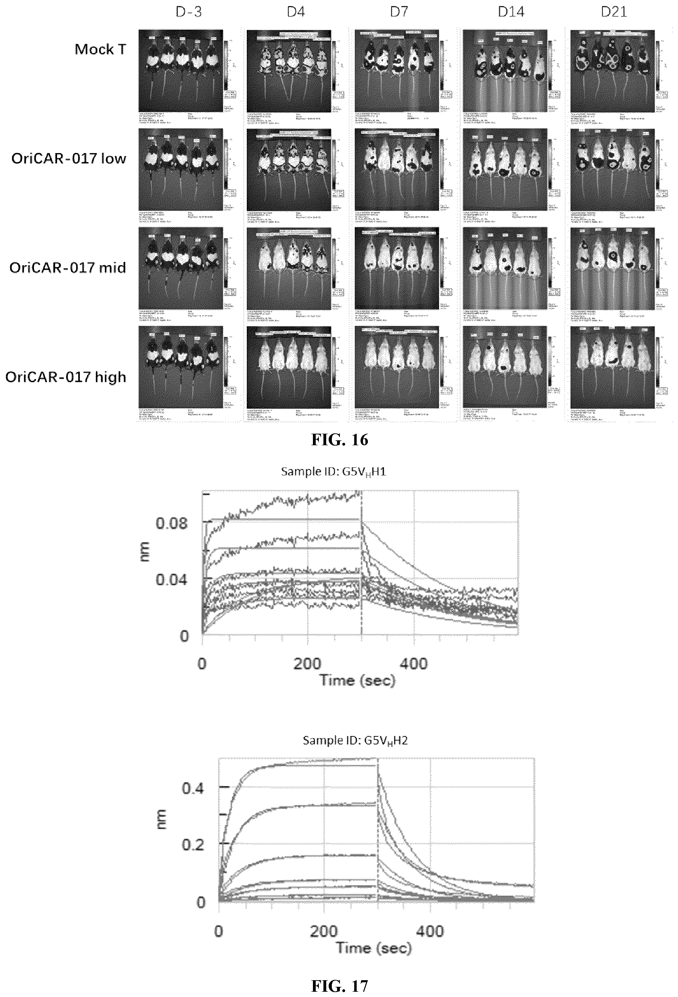

As shown in FIG. 16, compared with Mock T, different doses of OriCAR-017 cryopreserved cells all exhibit significant anti-tumor activity, greatly reducing the tumor load of the mice. Among them, the low-dose group exhibit an effective tumor-clearing effect on Day 7 after the refusion, while the OriCAR-017 medium- and high-dose groups exhibit significant tumor suppressing effect in a short term (on Day 4) after the refusion. In particular, the high-dose group can rapidly clear out the tumor and continue to suppress the tumor growth after the refusion. On Day 21, the high-dose group can achieve a tumor complete clearance rate up to 60%.

## Example 12. Clinical Study of OriCAR-017 CAR-T In Patients With Refractory/Recurrent Multiple Myeloma

A single-arm, open-label, phase I, multicenter, clinical study was conducted to determine the safety and effectiveness of the OriCAR-017 CAR-T cells in the treatment of human patients diagnosed as refractory or recurrent multiple myeloma ("r/r MM"). The clinical trial registration information is www.clinicaltrials.gov, and the identifier is NCT05016778.

In this study, the OriCAR-017 CAR-T cells derived from the autologous T cells of the patients were used to treat the patients with refractory/recurrent multiple myeloma. The CAR$^+$ T cells were administered to each patient by intravenous injection at doses of $1\times10^6$/kg, $3\times10^6$/kg, and $6\times10^6$/kg. From Day 1 to Day 30 of the study, the patients were monitored for their adverse events, and sampled for evaluation. All patients were followed up for at least 36 months after the CAR T administration. The study has a primary outcome of measuring the occurrence of treatment-related adverse events assessed by the Common Terminology Criteria for Adverse Events (CTCAE) v4 .0 within 1-30 days after the injection of the OriCAR-017 CAR-T cells; and a secondary outcome of evaluating the CAR-T-induced anti-myeloma response, for example, by measuring the abnormal immunoglobulin levels in serum and the number of multiple myeloma cells in the patient's bone marrow before and after the administration of the OriCAR-017 CAR-T cells. The efficacy goals of the study comprise the proportion of pathological complete response, 3-year disease-free survival rate and 3-year progression-free survival rate. This study was introduced in detail in 2022 ASCO Annual Meeting (https://ascopubs.org/doi/abs/10.1200/J CO.2022.40.16_suppl.8004). By April 30th, 2022, 12 patients were comprised in the study and received apheresis, and 10 patients had completed the OriCAR-017 CAR-T infusion, with the median age of 64 years old (within a range of 41-71 years old) and the median number of previous treatment lines of 5.5 lines (within a range of 3-17). Five (50.0%) patients had previously received anti-BCMA CAR-T therapy. No dose-limiting toxicity occurred. The most common adverse events (TEAEs) during the treatment are neutropenia, thrombocytopenia, leukopenia, lymphopenia, and anemia. No neurological toxicity was reported. Cytokine release syndrome (CRS) occurred in all patients, with 9 cases of grade 1 and 1 case of grade 2. After routine intervention (with Tozumab and steroids), all CRS were rapidly relieved. The median onset time was 2 days (within a range of 1-9 days) and the median duration was 6 days (within a range of 3-9 days). The objective remission rate (ORR) was 100%, the MRD was 100% negative, and the rate of complete remission / strict complete remission (CR/sCR) was 60%. The five patients previously receiving anti-BCMA CAR-T therapy can be evaluated for their efficacy; wherein two cases have reached sCR, two cases have reached VGPR, and one case has reached PR, specifically as shown in Tables 4 and 5.

Table 4 Patient Information at Enrollment

|  | $1\times10^6$/kg | $3\times10^6$/kg | $6\times10^6$/kg | Total |
|---|---|---|---|---|
|  | (N = 3) | (N = 4) | (N = 3) | (N= 10) |
| Age (years) |  |  |  |  |
| Case Number | 3 | 4 | 3 | 10 |
| Average (Standard Deviation) | 69.0 (3.46) | 53.5 (10.34) | 64.0 (3.61) | 61.3 (9.51) |
| Median | 71 | 54 | 63 | 64 |
| Q1 - Q3 | 65.0 - 71.0 | 45.5 - 61.5 | 61.0 - 68.0 | 58.0 - 68.0 |
| Minimum - Maximum | 65 - 71 | 41 - 65 | 61 - 68 | 41 - 71 |
| Age Grouping, n (%) |  |  |  |  |
| <65 years old | 0 | 3 (75.0) | 2 (66.7) | 5 (50.0) |
| ≥65 years old | 3 (100) | 1 (25.0) | 1 (33.3) | 5 (50.0) |
| Sex, n (%) |  |  |  |  |
| Male | 1 (33.3) | 2 (50.0) | 2 (66.7) | 5 (50.0) |
| Female | 2 (66.7) | 2 (50.0) | 1 (33.3) | 5 (50.0) |
| Nationality, n (%) |  |  |  |  |
| the Han Nationality | 3 (100) | 4 (100) | 3 (100) | 10 (100) |
| Other Nationality | 0 | 0 | 0 | 0 |

Table 5 Therapeutic Effect of OriCAR-017 CAR-T

|  | $1\times10^6$/kg | $3\times10^6$/kg | $6\times10^6$/kg | Total |
|---|---|---|---|---|
|  | (N = 3) | (N = 4) | (N = 3) | (N= 10) |
| Therapeutic Effect Evaluation, n (%) |  |  |  |  |
| sCR | 3 (100) | 3 (75.0) | 0 | 6 (60.0) |

(continued)

|  | 1×10⁶/kg | 3×10⁶/kg | 6×10⁶/kg | Total |
|---|---|---|---|---|
|  | (N = 3) | (N = 4) | (N = 3) | (N= 10) |
| Therapeutic Effect Evaluation, n (%) |  |  |  |  |
| CR | 0 | 0 | 0 | 0 |
| VGPR | 0 | 0 | 3 (100) | 3 (30.0) |
| PR | 0 | 1 (25.0) | 0 | 1 (10.0) |
| MR | 0 | 0 | 0 | 0 |
| SD | 0 | 0 | 0 | 0 |
| PD | 0 | 0 | 0 | 0 |
| ORR, n (%) | 3 (100) | 4 (100) | 3 (100) | 10 (100) |
| 95% CI [1] | 29.2, 100.0 | 39.8, 100.0 | 29.2, 100.0 | 69.2, 100.0 |
| DCR, n (%) | 3 (100) | 4 (100) | 3 (100) | 10 (100) |
| 95% CI [1] | 29.2, 100.0 | 39.8, 100.0 | 29.2, 100.0 | 69.2, 100.0 |

ORR = (number of CR + sCR + VGPR + PR cases) / (number of subject cases with evaluable therapeutic efficacy) × 100%.
DCR = (number of CR + sCR + VGPR + PR + MR cases) / (number of subject cases with evaluable therapeutic efficacy) × 100%.

The percentages were calculated based on the number of cases enrolled in each dose group in the subjects with evaluable therapeutic efficacy.

[1] The respective CIs on both sides were calculated based on the Clopper Pearson method.

**Example 13. Differential Epitope Binding Experiment of Two V$_H$H domains in GPRC5D CAR-T Cells**

The epitopes of GPRC5D V$_H$H domain in the OriCAR-017 CAR-T were identified. Exemplary bivalent GPRC5D CAR with two different anti-GPRC5D V$_H$H domains specifically binding to different epitopes of GPRC5D was constructed. The bivalent/dual-epitope CAR containing G5V$_H$H1 and G5V$_H$H2 was named as OriCAR-017, which is an exemplary dual-binding-domain GPRC5D double V$_H$H CAR listed in Table 2.

13.1 Determination of FORTEBIO Antibody

The G5V$_H$H1 and G5V$_H$H2 antibody sequences were cloned into an eukaryotic vector containing p3.4-hIgG1Fc of a human IgG1 Fc fragment (hIgG1Fc) sequence, facilitating the recombinant expression of GPRC5D V$_H$H-hIgG1Fc to give a recombinant protein which was then purified to determine the affinity of G5V$_H$H1-hIgG1Fc and G5V$_H$H2-hIgG1Fc to the affinity N-terminal peptide (Biotin) of GPRC5D. Briefly, the N-terminal peptide of human GPRC5D (>sp|Q9NZD1|1-27: MYKDCIESTGDYFLLCDAEGPWGIILE) protein was labeled with biotin (EZ-Link Sulfo-NHS-LC-Biotin, Pierce, 21327). By the biolayer interferometry (BLI) technique, the binding kinetics between antigen and antibody was analyzed using the molecular interaction analyzer fortebiooctetRED384 (PALL) (wherein both antigen and antibody were diluted with a PBS buffer containing 0.1% BSA and 0.02% Tween 20). The biotin-conjugated antigen with a concentration of 20 nM was immobilized to an SA sensor, and bound for 3 min; then bound with double-diluted antibody solution (G5V$_H$H1 and G5V$_H$H2 antibodies) for 5 min; and finally dissociated for 5 min. The obtained results were analyzed by Octet Data Analysis 9.0 software (fortebio) to calculate the binding strength between the antigen and the antibody, thereby giving the KD value, Ka (1/Ms) value and Kd (1/s) value. The results of binding affinity of G5V$_H$H1-hIgG1Fc and G5V$_H$H2-hIgG1Fc are shown in FIG. 17. The regression equation of G5V$_H$H1 antibody detection has a low goodness of fit, R2 (0.7914) for the sample data points, and the model is not credible. It can be seen that G5V$_H$H1 does not bind to the N-terminal peptide of GPRC5D, while the G5V$_H$H2 antibody binds to the N-terminal peptide with KD of 7.69E-08M; further indicating that there are differences between the epitopes of G5V$_H$H1 and G5V$_H$H2 binding to the GPRC5D target.

13.2 Competitive Binding Assay

The differential epitope binding of $G5V_HH1$ and $G5V_HH2$ was further demonstrated by cell-based competitive binding assay. Stable CHO cell lines (CHO-GPRC5D) that overexpress human GPRC5D were used for measurement. In brief, $G5V_HH1$ and $G5V_HH2$ were cloned into an eukaryotic vector containing p3.4-hIgG1Fc of human IgG1 Fc fragment (hIgG1Fc) sequence and an expression vector containing $6 \times$His tags, facilitating the recombinant expression and purification of GPRC5D $V_HH$-hIgG1Fc and GPRC5D $V_HH$-His to give $G5V_HH1$-hIgG1Fc, $G5V_HH2$-hIgG1Fc, $G5V_HH1$-His, $G5V_HH2$-His recombinant protein.

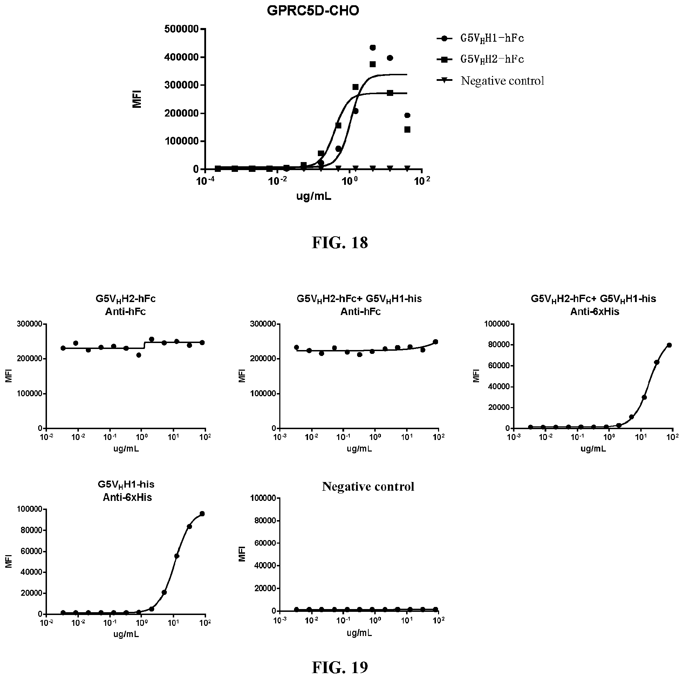

CHO-GPRC5D target cells were formulated with PBS buffer to a concentration of $1 \times 10^6$ cells/mL, and added into a 96-well sharp-bottom plate (corning 3894) with 30 $\mu$L in each well. The antibodies to be detected, $G5V_HH1$-hIgG1Fc and $G5V_HH2$-hIgG1Fc were formulated with PBS buffer to a concentration of 30 $\mu$g/mL, and subject to 3-fold dilution to form a gradient of 8 concentrations (with the minimum concentration of 0). The formulated antibodies with different concentrations were mixed well with the plated target cells at 30 $\mu$L/well, and incubated in a refrigerator at 4°C for 1 hr. 150 $\mu$L of buffer was added into each well, and centrifuged at 300 g for 5 min. The supernatant was then discarded, and the cells were additionally washed once. Goat-anti-human IgG (Fc-specific, ab98593) fluorescent secondary antibody was formulated with buffer at a ratio of 1:200, added into each well in an amount of 30 $\mu$L and mixed with the cells uniformly, and incubated in the refrigerator at 4°C for 30 min. 170 $\mu$L of buffer was added to each well, and centrifuged at 300 g for 5 min. The supernatant was then discarded and the cells were additionally washed once. 35 $\mu$L of buffer was added into each well, mixed well, and the mixture was detected by iQue Screener Flow Cytometer (available from IntelliCyt Company). The binding activity EC80 values of $G5V_HH1$-hIgG1Fc and $G5V_HH2$-hIgG1Fc were obtained according to the analysis results of the flow affinity binding experiment, as shown in FIG. 18.

The $G5V_HH2$-hIgG1Fc at an antibody concentration of 0.65 ng/mL corresponding to the EC80 was incubated with a total number of $3 \times 10^4$ CHO-GPRC5D cells for 1 h. The antibody to be detected, the $G5V_HH1$-His was formulated with PBS buffer to a concentration of 80 $\mu$g/mL, and subject to 3-fold dilution to form a gradient of 12 concentrations (with the minimum concentration of 0). Then, the serially diluted $G5V_HH1$-His recombinant antibody was added into the wells in the plate, and incubated at 4°C for additional 1 hr. After the incubation, 140 $\mu$L of buffer was added into each well, and centrifuged at 300 g for 5 min. The supernatant was then discarded, and the cells were additionally washed once. Goat-anti-human IgG (Fc-specific, ab98593) fluorescent secondary antibody was formulated with buffer at a ratio of 1:200, added into each well in an amount of 30 $\mu$L and mixed with the cells uniformly, and incubated in the refrigerator at 4°C for 30 min. 170 $\mu$L of buffer was added to each well, and centrifuged at 300 g for 5 min. The supernatant was then discarded, and the cells were additionally washed once. 35 $\mu$L of buffer was added into each well, mixed well, and the mixture was detected by iQue Screener Flow Cytometer (available from IntelliCyt Company). As an assay control, the binding of $G5V_HH1$-His to CHO-GPRC5D cells was detected in accordance with the same procedure as above, except using serial dilutions of $G5V_HH1$-His which were directly incubated with CHO-GPRC5D cells in the absence of $G5V_HH2$hIgG1Fc.

As shown in FIG. 19, under the saturated binding of $G5V_HH2$-hIgG1Fc to the cells, $G5V_HH1$-His still has an antibody gradient dependence, and has similar binding activity to that of $G5V_HH1$-His in the absence of $G5V_HH2$-hIgG1Fc, that is, the presence of $G5V_HH2$-hIgG1Fc does not affect the binding of $GSV_HH1$-His. Moreover, under the saturated binding of $G5V_HH2$-hIgG1Fc and the cells is, while $G5V_HH1$-His is additionally added to bind to the target cells, it is found that $G5V_HH2$-hIgG1Fc has a similar binding activity with that of $G5V_HH2$-hIgG1Fc in the absence of $GSV_HH1$-His, that is, the presence of $G5V_HH1$-His does not affect the binding of $GSV_HH1$-His. In summary, $G5V_HH1$ and $G5V_HH2$ have different epitopes binding to the GPRC5D target, which do not affect each other.

**Example 14. Cross-Binding Studies of CAR Antibody Sequence with Target Antigens of Different Species**

Stably transfected cells expressing human GPRC5D, cynomolgus monkey GPRC5D (SEQ ID NO: 36), mouse GPRC5D (SEQ ID NO: 37), human GPRC5D control subtype GPRC5A (SEQ ID NO: 38) were used to evaluate the binding of different anti-GPRC5D antibodies to human, cynomolgus monkey and mouse GPRC5D by flow cytometry. In brief, CHO-HuGPRC5D, CHO-CyGPRC5D, CHO-MuGPRC5D, and CHO-HuGPRC5A target cells were formulated with PBS buffer to concentration of $1 \times 10^6$ cells/mL, and added into a 96-well V-bottom plate with 30 $\mu$L in each well. The antibody to be detected, Double-$V_HH$ hFc, was formulated with PBS buffer to a concentration of 30 $\mu$g/mL, and subject to 3-fold dilution to form a gradient of 8 concentrations (with the minimum concentration of 0). The formulated antibodies with different concentrations were mixed well with the plated target cells at 30 $\mu$L/well, and incubated in a refrigerator at 4°C for 1 hr. 150 $\mu$L of buffer was added into each well, and centrifuged at 300 g for 5 min. The supernatant was then discarded, and the cells were additionally washed once. Dyna650-goat-anti-human IgG (Fc-specific, ab98593) fluorescent secondary antibody was formulated with buffer at a ratio of 1:200, added into each well in an amount of 30 $\mu$L and mixed with the cells uniformly, and incubated in the refrigerator at 4°C for 30 min. 170 $\mu$L of buffer was added to each well, and centrifuged at 300 g for 5 min. The supernatant was then discarded, and the cells were additionally washed once. 35 $\mu$L of buffer was added into each well, mixed well, and the mixture was detected by iQue Screener Flow Cytometer (available from IntelliCyt Company).

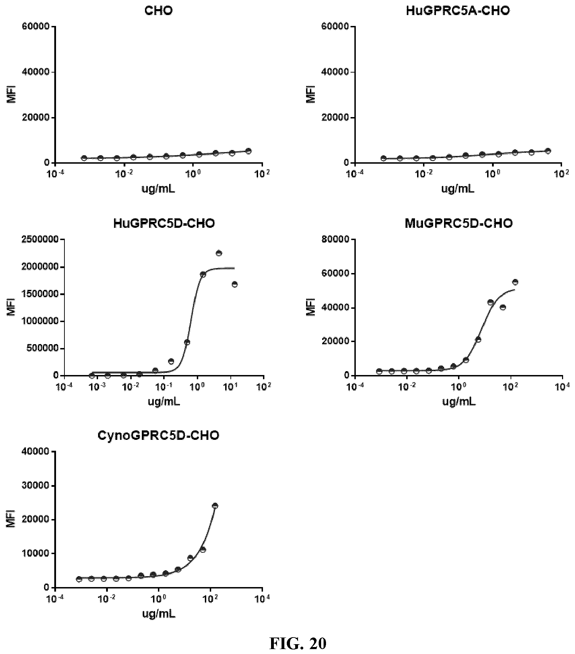

The results of the binding activity test by flow cytometry are shown in FIG. 20, and the binding properties are summarized in Table 6. It can be seen that the dual-binding-domain $V_H$H-hFc antibody shows that the dual-binding-domain $V_H$H-hFc antibody has strong binding activity to human GPRC5D, and relatively week binding activity to mouse GPRC5D and Cynomolgus monkey GPRC5D; no binding of the dual-binding-domain $V_H$H-hFc antibody to blank CHO cells was detected, and no binding of the dual-binding-domain $V_H$H-hFc antibody to CHO cells expressing GPRC5A which is a different subtype in the same family as human GPRC5D was detected, indicating that a high specificity of the dual-binding-domain $V_H$H-hFc antibody used in this product, which can reduce the risk of various off-target side effects.

Table 6 Summary of Cross-Binding Properties of Dual-Binding Domain $V_H$H-hFc Antibody with Target Antigens of Different Species

| Human GPRC5A | Human GPRC5D | Mouse GPRC5D | Cynomolgus monkey GPRC5D |
| --- | --- | --- | --- |
| - | ++ | + | + |
| Notes: - with no binding activity; + with relatively weak binding activity; ++ with strong binding activity. | | | |

The foregoing detailed description is provided by way of explanation and examples and is not intended to limit the scope of the appended claims. Various changes of the embodiments currently set forth in this application are obvious to those of ordinary skills in the art and are reserved within the scope of the appended claims and their equivalents.

**Claims**

1. A chimeric antigen receptor, comprising a first antigen binding domain and a second antigen binding domain, said first antigen binding domain targets a G protein-coupled receptor class C group 5 member D (GPRC5D) protein, and said second antigen binding domain targets the GPRC5D protein.

2. The chimeric antigen receptor of claim 1, wherein said first antigen binding domain and/or said second antigen binding domain comprises an antibody or an antigen binding fragment thereof.

3. The chimeric antigen receptor of claim 2, wherein said antigen binding fragment comprises Fab, Fab', F(ab)$_2$, an Fv fragment, F(ab')$_2$, scFv, di-scFv, VHH and/or dAb.

4. The chimeric antigen receptor of any one of claims 2-3, wherein said antigen binding fragment comprises a VHH.

5. The chimeric antigen receptor of any one of claims 1-4, wherein said first antigen binding domain comprises at least one CDR of an antibody heavy chain variable region VH, said VH comprises an amino acid sequence as set forth in any one of SEQ ID NO: 15, SEQ ID NO: 19, SEQ ID NO: 24 and SEQ ID NO: 30.

6. The chimeric antigen receptor of any one of claims 1-5, wherein said first antigen binding domain comprises a HCDR3, and said HCDR3 comprises an amino acid sequence as set forth in SEQ ID NO: 33.

7. The chimeric antigen receptor of any one of claims 1-6, wherein said first antigen binding domain comprises a HCDR3, and said HCDR3 comprises an amino acid sequence as set forth in any one of SEQ ID NO: 10, SEQ ID NO: 18, SEQ ID NO: 22 and SEQ ID NO: 27.

8. The chimeric antigen receptor of any one of claims 1-7, wherein said first antigen binding domain comprises a HCDR2, and said HCDR2 comprises an amino acid sequence as set forth in SEQ ID NO: 34 ($IX_1X_2X_3X_4GX_5T$, wherein $X_1$ is N or T, $X_2$ is S or W, $X_3$ is G or S, $X_4$ is D or G, and $X_5$ is N, S or T).

9. The chimeric antigen receptor of any one of claims 1-8, wherein said first antigen binding domain comprises a HCDR2, and said HCDR2 comprises an amino acid sequence as set forth in any one of SEQ ID NO: 9, SEQ ID NO: 17, SEQ ID NO: 21 and SEQ ID NO: 26.

10. The chimeric antigen receptor of any one of claims 1-9, wherein said first antigen binding domain comprises a HCDR1, and said HCDR1 comprises an amino acid sequence as set forth in SEQ ID NO: 35.

11. The chimeric antigen receptor of any one of claims 1-10, wherein said first antigen binding domain comprises a HCDR1, and said HCDR1 comprises an amino acid sequence as set forth in any one of SEQ ID NO: 8, SEQ ID NO: 16, SEQ ID NO: 20 and SEQ ID NO: 25.

12. The chimeric antigen receptor of any one of claims 1-11, wherein said first antigen binding domain comprises a HCDR1, a HCDR2 and a HCDR3, said HCDR1 comprises an amino acid sequence as set forth in SEQ ID NO: 35, said HCDR2 comprises an amino acid sequence as set forth in SEQ ID NO: 34 ($IX_1X_2X_3X_4GX_5T$, wherein $X_1$ is N or T, $X_2$ is S or W, $X_3$ is G or S, $X_4$ is D or G, and $X_5$ is N, S or T), and said HCDR3 comprises an amino acid sequence as set forth in SEQ ID NO: 33.

13. The chimeric antigen receptor of any one of claims 1-12, wherein said first antigen binding domain comprises a HCDR1, a HCDR2 and a HCDR3, and said HCDR1, HCDR2, HCDR3 comprise any one group of amino acid sequences selected from:

    (1) said HCDR1 comprises an amino acid sequence as set forth in SEQ ID NO: 8, said HCDR2 comprises an amino acid sequence as set forth in SEQ ID NO: 9, and said HCDR3 comprises an amino acid sequence as set forth in SEQ ID NO: 10;
    (2) said HCDR1 comprises an amino acid sequence as set forth in SEQ ID NO: 16, said HCDR2 comprises an amino acid sequence as set forth in SEQ ID NO: 17, and said HCDR3 comprises an amino acid sequence as set forth in SEQ ID NO: 18;
    (3) said HCDR1 comprises an amino acid sequence as set forth in SEQ ID NO: 20, said HCDR2 comprises an amino acid sequence as set forth in SEQ ID NO: 21, and said HCDR3 comprises an amino acid sequence as set forth in SEQ ID NO: 22; and
    (4) said HCDR1 comprises an amino acid sequence as set forth in SEQ ID NO: 25, said HCDR2 comprises an amino acid sequence as set forth in SEQ ID NO: 26, and said HCDR3 comprises an amino acid sequence as set forth in SEQ ID NO: 27.

14. The chimeric antigen receptor of any one of claims 10-13, wherein said first antigen binding domain comprises a H-FR1, a C-terminal of said H-FR1 is connected to an N-terminal of said HCDR1 directly or indirectly, and said H-FR1 comprises an amino acid sequence as set forth in SEQ ID NO: 11.

15. The chimeric antigen receptor of any one of claims 10-14, wherein said first antigen binding domain comprises a H-FR2, said H-FR2 is located between said HCDR1 and said HCDR2, and said H-FR2 comprises an amino acid sequence as set forth in any one of SEQ ID NO: 12, SEQ ID NO: 28 and SEQ ID NO: 23.

16. The chimeric antigen receptor of any one of claims 8-15, wherein said first antigen binding domain comprises a H-FR3, said H-FR3 is located between said HCDR2 and said HCDR3, and said H-FR3 comprises an amino acid sequence as set forth in SEQ ID NO: 13 or SEQ ID NO: 29.

17. The chimeric antigen receptor of any one of claims 6-16, wherein said first antigen binding domain comprises a H-FR4, an N-terminal of said H-FR4 is connected to a C-terminal of said HCDR3, and said H-FR4 comprises an amino acid sequence as set forth in SEQ ID NO: 14.

18. The chimeric antigen receptor of any one of claims 1-17, wherein said first antigen binding domain comprises a VH, and said VH comprises an amino acid sequence as set forth in any one of SEQ ID NO: 15, SEQ ID NO: 19, SEQ ID NO: 24 and SEQ ID NO: 30.

19. The chimeric antigen receptor of any one of claims 1-18, wherein said first antigen binding domain is a VHH, and said VHH comprises an amino acid sequence as set forth in any one of SEQ ID NO: 15, SEQ ID NO: 19, SEQ ID NO: 24 and SEQ ID NO: 30.

20. The chimeric antigen receptor of any one of claims 1-19, wherein said second antigen binding domain comprises at least one CDR of antibody heavy chain variable region VH, said VH comprises an amino acid sequence as set forth in any one of SEQ ID NO: 15, SEQ ID NO: 19, SEQ ID NO: 24 and SEQ ID NO: 30.

21. The chimeric antigen receptor of any one of claims 1-20, wherein said second antigen binding domain comprises a HCDR3, and said HCDR3 comprises an amino acid sequence as set forth in SEQ ID NO: 33.

22. The chimeric antigen receptor of any one of claims 1-21, wherein said second antigen binding domain comprises a HCDR3, and said HCDR3 comprises an amino acid sequence as set forth in any one of SEQ ID NO: 10, SEQ ID NO: 18, SEQ ID NO: 22 and SEQ ID NO: 27.

23. The chimeric antigen receptor of any one of claims 1-22, wherein said second antigen binding domain comprises a HCDR2, and said HCDR2 comprises an amino acid sequence as set forth in SEQ ID NO: 34 ($IX_1X_2X_3X_4GX_5T$, wherein $X_1$ is N or T, $X_2$ is S or W, $X_3$ is G or S, $X_4$ is D or G, and $X_5$ is N, S or T).

24. The chimeric antigen receptor of any one of claims 1-23, wherein said second antigen binding domain comprises a HCDR2, and said HCDR2 comprises an amino acid sequence as set forth in any one of SEQ ID NO: 9, SEQ ID NO: 17, SEQ ID NO: 21 and SEQ ID NO: 26.

25. The chimeric antigen receptor of any one of claims 1-24, wherein said second antigen binding domain comprises a HCDR1, and said HCDR1 comprises an amino acid sequence as set forth in SEQ ID NO: 35.

26. The chimeric antigen receptor of any one of claims 1-25, wherein said second antigen binding domain comprises a HCDR1, and said HCDR1 comprises an amino acid sequence as set forth in any one of SEQ ID NO: 8, SEQ ID NO: 16, SEQ ID NO: 20 and SEQ ID NO: 25.

27. The chimeric antigen receptor of any one of claims 1-26, wherein said second antigen binding domain comprises a HCDR1, a HCDR2 and a HCDR3, said HCDR1 comprises an amino acid sequence as set forth in SEQ ID NO: 35, said HCDR2 comprises an amino acid sequence as set forth in SEQ ID NO: 34 ($IX_1X_2X_3X_4GX_5T$, wherein $X_1$ is N or T, $X_2$ is S or W, $X_3$ is G or S, $X_4$ is D or G, and $X_5$ is N, S or T), and said HCDR3 comprises an amino acid sequence as set forth in SEQ ID NO: 33.

28. The chimeric antigen receptor of any one of claims 1-27, wherein said second antigen binding domain comprises a HCDR1, a HCDR2 and a HCDR3, and said HCDR1, HCDR2, HCDR3 comprise any one group of amino acid sequences selected from:

    (1) said HCDR1 comprises an amino acid sequence as set forth in SEQ ID NO: 8, said HCDR2 comprises an amino acid sequence as set forth in SEQ ID NO: 9, and said HCDR3 comprises an amino acid sequence as set forth in SEQ ID NO: 10;
    (2) said HCDR1 comprises an amino acid sequence as set forth in SEQ ID NO: 16, said HCDR2 comprises an amino acid sequence as set forth in SEQ ID NO: 17, and said HCDR3 comprises an amino acid sequence as set forth in SEQ ID NO: 18;
    (3) said HCDR1 comprises an amino acid sequence as set forth in SEQ ID NO: 20, said HCDR2 comprises an amino acid sequence as set forth in SEQ ID NO: 21, and said HCDR3 comprises an amino acid sequence as set forth in SEQ ID NO: 22; and
    (4) said HCDR1 comprises an amino acid sequence as set forth in SEQ ID NO: 25, said HCDR2 comprises an amino acid sequence as set forth in SEQ ID NO: 26, and said HCDR3 comprises an amino acid sequence as set forth in SEQ ID NO: 27.

29. The chimeric antigen receptor of any one of claims 25-28, wherein said second antigen binding domain comprises a H-FR1, a C-terminal of said H-FR1 is connected to an N-terminal of said HCDR1 directly or indirectly, and said H-FR1 comprises an amino acid sequence as set forth in SEQ ID NO: 11.

30. The chimeric antigen receptor of any one of claims 25-29, wherein said second antigen binding domain comprises a H-FR2, said H-FR2 is located between said HCDR1 and said HCDR2, and said H-FR2 comprises an amino acid sequence as set forth in any one of SEQ ID NO: 12, SEQ ID NO: 28 and SEQ ID NO: 23.

31. The chimeric antigen receptor of any one of claims 23-30, wherein said second antigen binding domain comprises a H-FR3, said H-FR3 is located between said HCDR2 and said HCDR3, and said H-FR3 comprises an amino acid sequence as set forth in SEQ ID NO: 13 or SEQ ID NO: 29.

32. The chimeric antigen receptor of any one of claims 21-31, wherein said second antigen binding domain comprises a H-FR4, an N-terminal of said H-FR4 is connected to a C-terminal of said HCDR3, and said H-FR4 comprises an amino acid sequence as set forth in SEQ ID NO: 14.

33. The chimeric antigen receptor of any one of claims 1-32, wherein said second antigen binding domain comprises a VH, and said VH comprises an amino acid sequence as set forth in any one of SEQ ID NO: 15, SEQ ID NO: 19, SEQ ID NO: 24 and SEQ ID NO: 30.

34. The chimeric antigen receptor of any one of claims 1-33, wherein said second antigen binding domain is a VHH, and said VHH comprises an amino acid sequence as set forth in any one of SEQ ID NO: 15, SEQ ID NO: 19, SEQ ID NO: 24 and SEQ ID NO: 30.

35. The chimeric antigen receptor of any one of claims 1-34, wherein said first antigen binding domain and said second antigen binding domain comprise a HCDR1, a HCDR2 and a HCDR3, respectively, and the sequences of said HCDR1, said HCDR2 and said HCDR3 of said first antigen binding domain and said second antigen binding domain are selected from any one group of amino acid sequences below:

(1) first antigen binding domain: said HCDR1 comprises an amino acid sequence as set forth in SEQ ID NO: 8, said HCDR2 comprises an amino acid sequence as set forth in SEQ ID NO: 9, and said HCDR3 comprises an amino acid sequence as set forth in SEQ ID NO: 10; second antigen binding domain: said HCDR1 comprises an amino acid sequence as set forth in SEQ ID NO: 8, said HCDR2 comprises an amino acid sequence as set forth in SEQ ID NO: 9, and said HCDR3 comprises an amino acid sequence as set forth in SEQ ID NO: 10;
(2) first antigen binding domain: said HCDR1 comprises an amino acid sequence as set forth in SEQ ID NO: 8, said HCDR2 comprises an amino acid sequence as set forth in SEQ ID NO: 9, and said HCDR3 comprises an amino acid sequence as set forth in SEQ ID NO: 10; second antigen binding domain: said HCDR1 comprises an amino acid sequence as set forth in SEQ ID NO: 16, said HCDR2 comprises an amino acid sequence as set forth in SEQ ID NO: 17, and said HCDR3 comprises an amino acid sequence as set forth in SEQ ID NO: 18;
(3) first antigen binding domain: said HCDR1 comprises an amino acid sequence as set forth in SEQ ID NO: 8, said HCDR2 comprises an amino acid sequence as set forth in SEQ ID NO: 9, and said HCDR3 comprises an amino acid sequence as set forth in SEQ ID NO: 10; second antigen binding domain: said HCDR1 comprises an amino acid sequence as set forth in SEQ ID NO: 20, said HCDR2 comprises an amino acid sequence as set forth in SEQ ID NO: 21, and said HCDR3 comprises an amino acid sequence as set forth in SEQ ID NO: 22;
(4) first antigen binding domain: said HCDR1 comprises an amino acid sequence as set forth in SEQ ID NO: 8, said HCDR2 comprises an amino acid sequence as set forth in SEQ ID NO: 9, and said HCDR3 comprises an amino acid sequence as set forth in SEQ ID NO: 10; second antigen binding domain: said HCDR1 comprises an amino acid sequence as set forth in SEQ ID NO: 25, said HCDR2 comprises an amino acid sequence as set forth in SEQ ID NO: 26, and said HCDR3 comprises an amino acid sequence as set forth in SEQ ID NO: 27;
(5) first antigen binding domain: said HCDR1 comprises an amino acid sequence as set forth in SEQ ID NO: 16, said HCDR2 comprises an amino acid sequence as set forth in SEQ ID NO: 17, and said HCDR3 comprises an amino acid sequence as set forth in SEQ ID NO: 18; second antigen binding domain: said HCDR1 comprises an amino acid sequence as set forth in SEQ ID NO: 8, said HCDR2 comprises an amino acid sequence as set forth in SEQ ID NO: 9, and said HCDR3 comprises an amino acid sequence as set forth in SEQ ID NO: 10;
(6) first antigen binding domain: said HCDR1 comprises an amino acid sequence as set forth in SEQ ID NO: 16, said HCDR2 comprises an amino acid sequence as set forth in SEQ ID NO: 17, and said HCDR3 comprises an amino acid sequence as set forth in SEQ ID NO: 18; second antigen binding domain: said HCDR1 comprises an amino acid sequence as set forth in SEQ ID NO: 16, said HCDR2 comprises an amino acid sequence as set forth in SEQ ID NO: 17, and said HCDR3 comprises an amino acid sequence as set forth in SEQ ID NO: 18;
(7) first antigen binding domain: said HCDR1 comprises an amino acid sequence as set forth in SEQ ID NO: 16, said HCDR2 comprises an amino acid sequence as set forth in SEQ ID NO: 17, and said HCDR3 comprises an amino acid sequence as set forth in SEQ ID NO: 18; second antigen binding domain: said HCDR1 comprises an amino acid sequence as set forth in SEQ ID NO: 20, said HCDR2 comprises an amino acid sequence as set forth in SEQ ID NO: 21, and said HCDR3 comprises an amino acid sequence as set forth in SEQ ID NO: 22;
(8) first antigen binding domain: said HCDR1 comprises an amino acid sequence as set forth in SEQ ID NO: 16, said HCDR2 comprises an amino acid sequence as set forth in SEQ ID NO: 17, and said HCDR3 comprises an amino acid sequence as set forth in SEQ ID NO: 18; second antigen binding domain: said HCDR1 comprises an amino acid sequence as set forth in SEQ ID NO: 25, said HCDR2 comprises an amino acid sequence as set forth in SEQ ID NO: 26, and said HCDR3 comprises an amino acid sequence as set forth in SEQ ID NO: 27;
(9) first antigen binding domain: said HCDR1 comprises an amino acid sequence as set forth in SEQ ID NO: 20, said HCDR2 comprises an amino acid sequence as set forth in SEQ ID NO: 21, and said HCDR3 comprises an amino acid sequence as set forth in SEQ ID NO: 22; second antigen binding domain: said HCDR1 comprises an amino acid sequence as set forth in SEQ ID NO: 8, said HCDR2 comprises an amino acid sequence as set forth in SEQ ID NO: 9, and said HCDR3 comprises an amino acid sequence as set forth in SEQ ID NO: 10;

(10) first antigen binding domain: said HCDR1 comprises an amino acid sequence as set forth in SEQ ID NO: 20, said HCDR2 comprises an amino acid sequence as set forth in SEQ ID NO: 21, and said HCDR3 comprises an amino acid sequence as set forth in SEQ ID NO: 22; second antigen binding domain: said HCDR1 comprises an amino acid sequence as set forth in SEQ ID NO: 16, said HCDR2 comprises an amino acid sequence as set forth in SEQ ID NO: 17, and said HCDR3 comprises an amino acid sequence as set forth in SEQ ID NO: 18;

(11) first antigen binding domain: said HCDR1 comprises an amino acid sequence as set forth in SEQ ID NO: 20, said HCDR2 comprises an amino acid sequence as set forth in SEQ ID NO: 21, and said HCDR3 comprises an amino acid sequence as set forth in SEQ ID NO: 22; second antigen binding domain: said HCDR1 comprises an amino acid sequence as set forth in SEQ ID NO: 20, said HCDR2 comprises an amino acid sequence as set forth in SEQ ID NO: 21, and said HCDR3 comprises an amino acid sequence as set forth in SEQ ID NO: 22;

(12) first antigen binding domain: said HCDR1 comprises an amino acid sequence as set forth in SEQ ID NO: 20, said HCDR2 comprises an amino acid sequence as set forth in SEQ ID NO: 21, and said HCDR3 comprises an amino acid sequence as set forth in SEQ ID NO: 22; second antigen binding domain: said HCDR1 comprises an amino acid sequence as set forth in SEQ ID NO: 25, said HCDR2 comprises an amino acid sequence as set forth in SEQ ID NO: 26, and said HCDR3 comprises an amino acid sequence as set forth in SEQ ID NO: 27;

(13) first antigen binding domain: said HCDR1 comprises an amino acid sequence as set forth in SEQ ID NO: 25, said HCDR2 comprises an amino acid sequence as set forth in SEQ ID NO: 26, and said HCDR3 comprises an amino acid sequence as set forth in SEQ ID NO: 27; second antigen binding domain: said HCDR1 comprises an amino acid sequence as set forth in SEQ ID NO: 8, said HCDR2 comprises an amino acid sequence as set forth in SEQ ID NO: 9, and said HCDR3 comprises an amino acid sequence as set forth in SEQ ID NO: 10;

(14) first antigen binding domain: said HCDR1 comprises an amino acid sequence as set forth in SEQ ID NO: 25, said HCDR2 comprises an amino acid sequence as set forth in SEQ ID NO: 26, and said HCDR3 comprises an amino acid sequence as set forth in SEQ ID NO: 27; second antigen binding domain: said HCDR1 comprises an amino acid sequence as set forth in SEQ ID NO: 16, said HCDR2 comprises an amino acid sequence as set forth in SEQ ID NO: 17, and said HCDR3 comprises an amino acid sequence as set forth in SEQ ID NO: 18;

(15) first antigen binding domain: said HCDR1 comprises an amino acid sequence as set forth in SEQ ID NO: 25, said HCDR2 comprises an amino acid sequence as set forth in SEQ ID NO: 26, and said HCDR3 comprises an amino acid sequence as set forth in SEQ ID NO: 27; second antigen binding domain: said HCDR1 comprises an amino acid sequence as set forth in SEQ ID NO: 20, said HCDR2 comprises an amino acid sequence as set forth in SEQ ID NO: 21, and said HCDR3 comprises an amino acid sequence as set forth in SEQ ID NO: 22; and

(16) first antigen binding domain: said HCDR1 comprises an amino acid sequence as set forth in SEQ ID NO: 25, said HCDR2 comprises an amino acid sequence as set forth in SEQ ID NO: 26, and said HCDR3 comprises an amino acid sequence as set forth in SEQ ID NO: 27; second antigen binding domain: said HCDR1 comprises an amino acid sequence as set forth in SEQ ID NO: 25, said HCDR2 comprises an amino acid sequence as set forth in SEQ ID NO: 26, and said HCDR3 comprises an amino acid sequence as set forth in SEQ ID NO: 27.

36. The chimeric antigen receptor of any one of claims 1-35, wherein said first antigen binding domain and said second antigen binding domain target the same epitope of GPRC5D.

37. The chimeric antigen receptor of any one of claims 1-36, wherein said first antigen binding domain and said second antigen binding domain comprise the same or different amino acid sequences.

38. The chimeric antigen receptor of any one of claims 1-37, wherein said first antigen binding domain and said second antigen binding domain are connected directly or indirectly.

39. The chimeric antigen receptor of any one of claims 1-38, wherein said first antigen binding domain and said second antigen binding domain are connected by a linker.

40. The chimeric antigen receptor of claim 39, wherein said linker comprises an amino acid sequence of (GGGGS)n, wherein said n is any positive integer from 1-10.

41. The chimeric antigen receptor of claim 39, wherein said linker comprises an amino acid sequence of (EAAAK)n, wherein said n is any positive integer from 1-10.

42. The chimeric antigen receptor of any one of claims 1-40, comprising a costimulatory signal region, wherein said costimulatory signal region comprises an intracellular costimulatory signal region derived from one or more proteins selected from a group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88.

43. The chimeric antigen receptor of claim 41, wherein said costimulatory signal region is an intracellular costimulatory signal region derived from 4-1BB.

44. The chimeric antigen receptor of any one of claims 42-43, wherein said costimulatory signal region comprises an amino acid sequence as set forth in SEQ ID NO: 4.

45. The chimeric antigen receptor of any one of claims 1-44, comprising an intracellular signaling domain, said intracellular signaling domain comprises an intracellular signal region derived from one or more proteins selected from a group consisting of: CD3ζ, CD3δ, CD3γ, CD3ε, CD79a, CD79b, FcεRIγ, FcεRIβ, FcγRIIa, bovine leukemia virus gp30, Epstein-Barr virus (EBV) LMP2A, simian immunodeficiency virus PBj 14 Nef, Kaposi's sarcoma herpes virus (HSKV), DAP10, DAP-12, and a domain at least containing one ITAM.

46. The chimeric antigen receptor of claim 45, wherein said intracellular signaling domain is a signaling domain derived from CD3ζ.

47. The chimeric antigen receptor of any one of claims 45-46, wherein said intracellular signaling domain comprises an amino acid sequence as set forth in SEQ ID NO: 5.

48. The chimeric antigen receptor of any one of claims 1-47, comprising a transmembrane region, said transmembrane region comprises a transmembrane domain derived from one or more proteins selected from a group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

49. The chimeric antigen receptor of claim 48, wherein said transmembrane region is a transmembrane region derived from CD8.

50. The chimeric antigen receptor of any one of claims 48-49, wherein said transmembrane region comprises an amino acid sequence as set forth in SEQ ID NO: 3.

51. The chimeric antigen receptor of any one of claims 1-50, further comprising a hinge region, said hinge region comprises a hinge region derived from one or more proteins selected from a group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

52. The chimeric antigen receptor of claim 51, wherein said hinge region is a hinge region derived from CD8.

53. The chimeric antigen receptor of any one of claims 51-52, wherein said hinge region comprises an amino acid sequence as set forth in SEQ ID NO: 2.

54. The chimeric antigen receptor of any one of claims 1-53, further comprising a low-density lipoprotein receptor-related protein or a fragment thereof.

55. The chimeric antigen receptor of claim 54, wherein said low-density lipoprotein receptor-related protein or the fragment thereof is located at a C terminal of said intracellular signal region.

56. The chimeric antigen receptor of any one of claims 54-55, wherein said low-density lipoprotein receptor-related protein or the fragment thereof comprises one or more selected from a group consisting of: low-density lipoprotein receptor-related proteins 1-12 and functional fragments thereof.

57. The chimeric antigen receptor of any one of claims 54-56, wherein said low-density lipoprotein receptor-related protein or the fragment thereof is low-density lipoprotein receptor-related proteins 5 and/or 6 or the fragments thereof.

58. The chimeric antigen receptor of any one of claims 54-57, wherein said low-density lipoprotein receptor-related protein or the fragment thereof comprises an amino acid sequence as set forth in SEQ ID NO: 7.

59. The chimeric antigen receptor of any one of claims 1-58, further comprising a signal peptide.

60. The chimeric antigen receptor of claim 59, wherein said signal peptide is derived from a signal peptide of CD8 protein.

61. The chimeric antigen receptor of any one of claims 59-60, wherein said signal peptide comprises an amino acid sequence as set forth in SEQ ID NO: 1.

62. One or more isolated nucleic acid molecules, encoding the chimeric antigen receptor of any one of claims 1-61.

63. The nucleic acid molecule of claim 62, further comprising a promoter.

64. The nucleic acid molecule of claim 63, wherein said promoter is a constitutive promoter.

65. The nucleic acid molecule of any one of claims 63-64, wherein said promoter is an EF1α promoter.

66. A vector, comprising the nucleic acid molecule of any one of claims 62-65.

67. The vector of claim 66, which is a viral vector.

68. The vector of any one of claims 66-67, which is a lentiviral vector.

69. A cell, comprising the chimeric antigen receptor of any one of claims 1-61, the nucleic acid molecule of any one of claims 62-65, and/or the vector of any one of claims 66-68.

70. The cell of claim 69, which is an immune effector cell.

71. The cell of any one of claims 69-70, comprising T cells, B cells, natural killer cells (NK cells), macrophages, NKT cells, monocytes, dendritic cells, granulocytes, lymphocytes, leukocytes, peripheral blood mononuclear cells, embryonic stem cells, lymphoid progenitor cells and/or pluripotent stem cells.

72. The cell of any one of claims 69-71, which is a T cell.

73. A method for preparing the chimeric antigen receptor of any one of claims 1-61, comprising culturing the cell of any one of claims 69-72 under a condition enabling the expression of the chimeric antigen receptor of any one of claims 1-61.

74. A method for preparing a modified immune effector cell, comprising introducing the vector of any one of claims 66-68 into said immune effector cell.

75. A pharmaceutical composition, comprising the chimeric antigen receptor of any one of claims 1-61, the nucleic acid molecule of any one of claims 62-65, the vector of any one of claims 66-68, and/or the cell of any one of claims 69-72, as well as optionally a pharmaceutically acceptable carrier.

76. Use of the chimeric antigen receptor of any one of claims 1-61, the nucleic acid molecule of any one of claims 62-65, the vector of any one of claims 66-68, the cell of any one of claims 69-72, and/or the pharmaceutical composition of claim 75 in the manufacture of a medicament for preventing, treating and/or alleviating a disease and/or a disorder.

77. The use of claim 76, wherein said disease and/or said disorder comprises a disease and/or a disorder associated with the abnormal expression of GPRC5D.

78. The use of claim 77, wherein said disease and/or said disorder associated with the abnormal expression of GPRC5D comprises a tumor.

79. The use of claim 78, wherein said tumor comprises a solid tumor.

80. The use of claim 78, wherein said tumor comprises a non-solid tumor.

81. The use of any one of claims 79-80, wherein said tumor comprises a hematological tumor and/or lymphoma.

82. The use of any one of claims 78-81, wherein said tumor comprises myeloma.

83. The use of claim 82, wherein said myeloma comprises refractory/relapse multiple myeloma.

84. A method for preventing, treating and/or alleviating a disease and/or a disorder, comprising administering to a subject in need thereof the cell of any one of claims 69-72, and/or the pharmaceutical composition of claim 75.

85. The method of claim 84, wherein said disease and/or said disorder comprises a disease and/or a disorder associated with the abnormal expression of GPRC5D.

86. The method of claim 85, wherein said disease or said disorder associated with the abnormal expression of GPRC5D comprises a tumor.

87. The method of claim 86, wherein said tumor comprises a solid tumor.

88. The method of claim 86, wherein said tumor comprises a non-solid tumor.

89. The method of any one of claims 87-88, wherein said tumor comprises a hematological tumor and/or lymphoma.

90. The method of any one of claims 87-89, wherein said tumor comprises myeloma.

91. The method of claim 90, wherein said myeloma comprises refractory/relapse multiple myeloma.

FIG. 1

FIG. 2A

FIG. 2B

**FIG. 3**

**FIG. 4A**

**FIG. 4B**

FIG. 5A

Effector cells: Target cells

FIG. 5B

FIG. 6A

FIG. 6A

FIG. 6B

FIG. 6A

FIG. 6B

**FIG. 6B**

**FIG. 7A**

**FIG. 7B**

**FIG. 8**

**FIG. 9**

CD25-PE          CD69-APC

**FIG. 10**

FIG. 11

FIG. 12

FIG. 13

Injection of 1×10⁷ MM.1S cells  Transfusion of CAR-T cells  Detection of T cells and cytokines

NSG    Day 14    Day 0    Day 7    Day 14    Day 21    Day 28

Measuring tumor size three times a week

**FIG. 14**

Secretion of IFN-γ factor

**FIG. 15A**

Change in body weight of mice

After transfusion of CAR-T

**FIG. 15B**

**FIG. 15C**

**FIG. 15D**

**FIG. 16**

Sample ID: G5V$_H$H1

Sample ID: G5V$_H$H2

**FIG. 17**

**FIG. 18**

**FIG. 19**

**FIG. 20**

| **INTERNATIONAL SEARCH REPORT** | International application No. |
|---|---|
| | **PCT/CN2023/071869** |

**A. CLASSIFICATION OF SUBJECT MATTER**

C07K 16/46(2006.01)i;C12N 15/13(2006.01)i;A61K39/395(2006.01)i;A61P35/00(2006.01)i

According to International Patent Classification (IPC) or to both national classification and IPC

**B. FIELDS SEARCHED**

Minimum documentation searched (classification system followed by classification symbols)

IPC: C07K; C12N; A61K; A61P

Documentation searched other than minimum documentation to the extent that such documents are included in the fields searched

Electronic data base consulted during the international search (name of data base and, where practicable, search terms used)

CNABS, DWPI, SIPOABS, CNKI, NCBI, ISI Web of Science: 嵌合抗原受体, 蛋白偶联受体, 重链, 纳米, 抗体, 串联, 二聚, 互补, chimeric antigen receptor, CAR, G protein-coupled receptor, GPRC5D, heavy chain, antibody, vhh, nanobody, tandem, dimer, biparatopic

**C. DOCUMENTS CONSIDERED TO BE RELEVANT**

| Category* | Citation of document, with indication, where appropriate, of the relevant passages | Relevant to claim No. |
|---|---|---|
| Y | CN 113597433 A (JANSSEN BIOTECHNOLOGY, INC.) 02 November 2021 (2021-11-02) entire document, in particular claims, and abstract | 1-91 |
| Y | Peter Bannas, et al. "Nanobodies and Nanobody-Based Human Heavy Chain Antibodies As Antitumor Therapeutics" *Frontiers in Immunology,* Vol. 8, 22 November 2017 (2017-11-22), entire document, in particular page 5, right column, the second-to-last paragraph, page 9, left column, paragraph 2, and abstract | 1-91 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **07 February 2023** | **20 February 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

| International application No. |
| --- |
| **PCT/CN2023/071869** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2023/071869**

| Patent document cited in search report | | | Publication date (day/month/year) | Patent family member(s) | | | Publication date (day/month/year) |
|---|---|---|---|---|---|---|---|
| CN | 113597433 | A | 02 November 2021 | CA | 3127025 | A1 | 23 July 2020 |
| | | | | JP | 2022518708 | A | 16 March 2022 |
| | | | | CO | 2021010075 | A2 | 19 August 2021 |
| | | | | EA | 202191939 | A1 | 12 October 2021 |
| | | | | IL | 284744 | A | 31 August 2021 |
| | | | | US | 2020231686 | A1 | 23 July 2020 |
| | | | | ECSP | 21060675 | A | 30 September 2021 |
| | | | | EP | 3911677 | A1 | 24 November 2021 |
| | | | | AU | 2020209257 | A1 | 05 August 2021 |
| | | | | WO | 2020148677 | A1 | 23 July 2020 |
| | | | | UY | 38546 | A | 31 July 2020 |
| | | | | CL | 2021001883 | A1 | 21 January 2022 |
| | | | | KR | 20210116562 | A | 27 September 2021 |
| | | | | TW | 202043263 | A | 01 December 2020 |
| | | | | SG | 11202107708 | XA | 30 August 2021 |
| | | | | PE | 20211977 | A1 | 05 October 2021 |
| | | | | AR | 119676 | A1 | 05 January 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107428829 A

- US 10633426 B2

**Non-patent literature cited in the description**

- *ASCO Annual Meeting*, 2022, https://ascopubs. org/doi/abs/10.1200/JCO.2022.40.16_suppl.8004